# EUROPÄISCHE PATENTSCHRIFT

(11) **EP 3 235 420 B1**
(45) Veröffentlichungstag und Bekanntmachung des Hinweises auf die Patenterteilung: **30.10.2019**
(21) Anmeldenummer: 17166648.0
(22) Anmeldetag: 13.04.2017
(51) Int. Cl.: A61B 1/247, A61C 7/14

(54) **INSTRUMENT FÜR ZAHNÄRZTLICHE ZWECKE, INSBESONDERE ZUR POSITIONIERUNG VON BRACKETS AN ZÄHNEN**
INSTRUMENT FOR DENTAL PURPOSES, IN PARTICULAR FOR POSITIONING OF BRACKETS ON TEETH
INSTRUMENT À DES FINS DENTAIRES, EN PARTICULIER DESTINÉ AU POSITIONNEMENT DE BRACKETS SUR DES DENTS

(30) Priorität: 22.04.2016 DE 102016107562
(43) Veröffentlichungstag der Anmeldung: 25.10.2017
(73) Patentinhaber: Technische Universität Dresden, 01069 Dresden (DE)
(72) Erfinder: Seeliger, Julia, 01307 Dresden (DE); Gedrange, Tomasz, 01219 Dresden (DE); Kondylis, Eleftherios, 45881 Gelsenkirchen (DE)
(74) Vertreter: Riechelmann & Carlsohn Patentanwälte PartG mbB

(56) Entgegenhaltungen:
- CN-U- 202 069 707
- CN-U- 205 041 542
- US-A- 5 076 784
- US-A1- 2004 081 935
- US-A1- 2008 166 677

## Beschreibung

Die Erfindung betrifft ein kieferorthopädisches Instrument, das insbesondere für die Positionierung von Brackets an Zähnen verwendet werden kann.

Um die Zahnstellung zu korrigieren, werden festsitzende Multibracket-Apparaturen verwendet, bei denen je ein Bracket auf einen Zahn geklebt wird. In einen horizontal verlaufenden Slot in der Mitte des Brackets wird ein Bogen einligiert, an dem die Zähne entlang bewegt werden können. Dieser Slot wird im Folgenden auch als Bracket-Slot bezeichnet. Das Platzieren der Brackets sollte so genau wie möglich erfolgen, damit eine optimale Behandlung durchgeführt werden kann. Um die Angulation des Zahnes korrekt einzustellen, wird das Bracket so auf dem Zahn positioniert, dass die senkrechte auf dem Bracket markierte Mittellinie exakt durch die Längsachse des Zahnes verläuft. Dies wird in der Regel mit einer Sonde, einem Scaler, einem Heidemann-Spatel (siehe DE 1 728 891 U) oder einem Modellierinstrument für den Dentalbereich durchgeführt (siehe beispielsweise DE 20 2014 009 183 U1). Dabei erfolgt das Platzieren der Brackets in der Regel in mesio-distaler Richtung in der Mitte der klinischen Krone des Zahnes. Dazu wird mit einem Mundspiegel der Zahn von okklusal betrachtet. Typische zahnärztliche Mundspiegel werden beispielsweise in DE 2 323 631 OS beschrieben und weisen einen stabförmigen Griff auf, an dem ein Spiegel angebracht ist (siehe beispielsweise 297 95 997 U1). Je nach Behandlungskonzept und Zahngattung wird ein Bracket mit einem festgelegten Abstand zur Inszisalkante positioniert. Dabei erleichtern Messlehren mit einer 0,5 mm-Skala das Finden der korrekten Position. In der Regel befindet sich das Bracket somit auch in der Vertikalen in der Mitte der klinischen Krone.

Zum Kleben eines Brackets auf einen Zahn sind zunächst vorbereitende Maßnahmen durchzuführen. Dazu wird, nach vorangegangener Zahnreinigung und Trockenlegung, bei Anwendung der konventionellen Konditionierung die Zahnoberfläche mit Phosphorsäure konditioniert. Die Phosphorsäure wird dann abgespült, und anschließend wird die geätzte Zahnoberfläche trocken gelegt. Auf die geätzte, trockene Zahnoberfläche wird dann ein dünner Film eines Haftvermittlers aufgetragen. Das Bracket wird von einer ersten Person, in der Regel einer Zahnarzthelferin, mit einem Adhäsiv beschichtet, in eine spezielle Klemmpinzette eingespannt und einer zweiten Person, dem Behandler, angereicht.

Der Behandler setzt das Bracktet auf die Zahnoberfläche. Nun findet die exakte Positionierung des Brackets statt. Mit Hilfe eines kleinen Heidemann-Spatels, einer Sonde oder eines Scalers werden die Angulation des Brackets korrigiert und Überschüsse des Adhäsivs entfernt. Mit Hilfe eines Messkreuzes oder eines Bracketpositionieres wird die vertikale Höhe des Brackets auf dem Zahn gemessen und die Bracketposition entsprechend verändert. Zur Messung der vertikalen Höhe des Brackets wird in der Regel der Abstand vom Bracket-Slot bis zur Inzisalkante bzw. Höckerspitze oder der Abstand von der okklusalen Bracketkante bis zur Inzisalkante bzw. Höckerspitze bestimmt.

Typischerweise werden diese Abstände in Millimeter gemessen, wobei häufig eine Skalierung von 3,5 mm, 4,0 mm, 4,5 mm oder 5,0 mm verwendet wird. Die Ausrichtung des Brackets in Bezug auf dessen vertikale Höhe wird im Folgenden auch als vertikale Positionierung bezeichnet.

Im Anschluss an die vertikale Positionierung des Brackets auf der Zahnoberfläche wird, um die mesio-distale Position des Brackets exakt bestimmen zu können, anschließend mit dem Mundspiegel von okklusal geprüft, ob das auf der vestibulären Kronenoberfläche befindliche Bracket in der Mitte des Zahnes vorzufinden ist. Die Nachkorrektur erfolgt entweder mit dem Heidemann-Spatel, der Sonde oder dem Scaler. Das exakte Prüfen der mesio-distalen Position des Brackets und die Anzahl der Nachkorrekturen hängen von der Erfahrung des Behandlers ab und werden so oft wiederholt, bis das Bracktet richtig positioniert ist. Die Ausrichtung des Brackets in Bezug auf dessen mesio-distale Position wird im Folgenden auch als horizontale Positionierung bezeichnet.

Alle Instrumente, die zum Anbringen und zur Positionierung eines Brackets am Zahn erforderlich sind, werden vor jedem Arbeitsschritt von der Zahnarzthelferin angereicht. Der Wechsel zwischen den erforderlichen Instrumenten, also Messkreuz/Bracketpositionierer, Heidemann-Spatel/Sonde/Scaler und Spiegel, ist zeitraubend, selbst dann, wenn die erste und zweite Person sehr erfahren sind.

Aus CN 205 041 542 U ist ein zahnärztliches Instrument bekannt, das zum "Anbringen von Brackets an Zähnen" verwendet werden soll. Das Instrument soll es ermöglichen, gleichzeitig die Dentalkronenform und die Bracketposition aus einer dreidimensionalen Richtung zu beobachten. Das Instrument weist eine Spiegeloberfläche auf, auf der ein Positionierungslineal angeordnet ist.

Aufgabe der Erfindung ist es, die Nachteile nach dem Stand der Technik zu beseitigen. Es soll insbesondere ein Instrument für zahnärztliche Zwecke angegeben werden, dass eine exakte Positionierung von Brackets an Zähnen, insbesondere eine exakte vertikale und horizontale Positionierung ermöglicht.

Diese Aufgabe wird durch die Merkmale des Anspruchs 1 gelöst. Zweckmäßige Ausgestaltungen der Erfindungen ergeben sich aus den Merkmalen der Unteransprüche.

Nach Maßgabe der Erfindung ist ein Instrument für zahnärztliche Zwecke, insbesondere zur Positionierung von Brackets an Zähnen, vorgesehen. Das Instrument weist einen Spiegel und ein Positionierungselement zur Bestimmung und Veränderung der Position des Brackets am Zahn auf. Das Instrument weist einen Griff auf. Der Spiegel und das Positionierungselement sind am selben Ende des Griffes angeordnet. Das Positionierungselement weist ein erstes Segment und ein zweites Segment auf. Die Haupterstreckungsrichtung des zweiten Segmentes verläuft annähernd parallel zur Längsachse des Griffes.

Das Instrument ersetzt die bisher für die Positionierung eines Brackets auf einem Zahn erforderlichen Werkzeuge, weil es deren Funktionen in vorteilhafter Weise vereint. Das Instrument kann insbesondere die folgenden Instrumente ersetzen:
(i) Instrumente zur Korrektur der Angulation, insbesondere Heidemann-Spatel, Sonden und Scaler;
(ii) Instrumente zur vertikalen Positionierung des Brackets am Zahn, insbesondere Messkreuze und Bracketpositionierer;
(iii) Instrumente zur horizontalen Positionierung des Brackets am Zahn, insbesondere Sonden, Heidemann-Spatel, Scaler und separate Mundspiegel.

Der Nutzen des erfindungsgemäßen Instrumentes liegt nicht nur in der Kombination von Positionierelement und Spiegel in einem Instrument. Das erfindungsgemäße Instrument hat zum Vorteil, dass mit ihm die Einzelschritte während der Bracketpositionierung erleichtert und beschleunigt werden können. Der Wechsel zwischen Spiegel und Positionierungshilfe nimmt weniger Zeit in Anspruch, da auf das mehrfache separate Anreichen der Instrumente durch die Zahnarzthelferin verzichtet werden kann. Gleichzeitig sinkt die Fehleranfälligkeit, weil der zeitliche Abstand zwischen der vertikalen und der horizontalen Positionierung sinkt und unbeabsichtigte Veränderungen der Position des Brackets auf dem Zahn sofort bemerkt werden können. Die zweite Person hat kontinuierlich den Behandlungsort im Blick und ist nicht mit der Interaktion mit der ersten Person, dem korrekten Fassen der angereichten Instrumente und der erneuten Führung der Instrumente zum Behandlungsort beschäftigt.

Zweckmäßigerweise weist das erfindungsgemäße Instrument einen Griff auf. Der Spiegel und das Positionierungselement sind dabei vorzugsweise am selben Ende des Griffes, dem Arbeitsende, angeordnet. Der Spiegel ist bevorzugt zur Haupterstreckungsrichtung des Griffes abgewinkelt angeordnet. Die Haupterstreckungsrichtung des Griffes ist bei einem zylinderförmigen oder annähernd zylinderförmigen Grundkörper des Griffs die Längsachse des Grundkörpers. Der Griff kann beispielsweise wie der Griff aus dem Stand der Technik bekannter zahnmedizinischer Mundspiegel gestaltet sein. Der Griff besteht vorzugsweise aus rostfreiem Edelstahl.

Es kann vorgesehen sein, dass das Positionierungselement eine Längenmesseinrichtung aufweist. Die Längenmesseinrichtung kann zur Bestimmung der Position des Brackets am Zahn eingesetzt werden, nachdem der Behandler das Bracket auf die Zahnoberfläche eines Zahns aufgesetzt hat. In der Regel wird dabei das Bracket in mesio-distaler Richtung in der Mitte der klinischen Krone des Zahnes aufgesetzt, womit eine grobe Vorpositionierung des Brackets auf der Zahnoberfläche vorgenommen wird. Mittels des Positionierungselementes kann dann die endgültige Positionierung vorgenommen werden. Die Längenmesseinrichtung kann insbesondere zur vertikalen Positionierung des Brackets am Zahn verwendet werden. Mittels des Positionierungselementes kann der Behandler den Abstand vom Bracket-Slot bis zur okklusalen Kante des Zahns oder den Abstand von der okklusalen Bracketkante bis zur okklusalen Kante des Zahns bestimmen. Die Längenmesseinrichtung kann eine Skale aufweisen, mit deren Hilfe diese Abstände gemessen werden können. Beispielsweise kann als Skale eine Skalierung mit Kennzeichnung von 0,5- oder 1-mm-Messabständen, gemessen von einer Nulllinie vorgesehen sein. Alternativ oder zusätzlich kann die Skale Messabstände von 3,0 mm, 3,5 mm, 4,0 mm, 4,5 mm oder 5,0 mm, gemessen vom einer Nulllinie, aufweisen. Die Skale kann in das Positionierungselement eingraviert sein. Die Messabstände können farbig hervorgehoben sein. Die Nulllinie kann dabei an einem Punkt oder einer Kante des Positionierungselementes liegen. Der Punkt oder die Kante kann vom Behandler dann am Bracketslot positioniert werden, wenn er mit Hilfe des erfindungsgemäßen Instrumentes die vertikale Positionierung des Brackets, aber auch die horizontale Positionierung des Brackets oder beides vornimmt.

Die Längenmesseinrichtung kann im Falle eines flachen Positionierungselementes an einer Kante, beispielsweise einer Längskante, oder auf einer oder beiden Flächenseiten des Positionierungselementes ausgebildet sein.

Das Positionierungselement kann eine Kontakteinrichtung zur Kontaktierung des Brackets und zur Veränderung der Position des Brackets am Zahn aufweisen. Die Kontakteinrichtung kann eine Kante, eine Spitze oder eine abgrundete Stirnseite des Positionierungselementes sein. Beispielsweise kann das Positionierungselement die Form des Werkzeuges einer Sonde, eines Scalers oder eines Heidemann-Spatels haben. Im Sinne der vorliegenden Erfindung wird unter dem Werkzeug einer Sonde, eines Scalers oder eines Heidemann-Spatels der Teil dieser Instrumente verstanden, der unmittelbar zur Manipulation eines Brackets verwendet werden kann, also nicht der Griff und, falls vorhanden, der Ansatz zwischen Griff und dem Werkzeug. Vorzugsweise hat das Positionierungselement die Form einer Sonde oder eines Heidemann-Spatels. Im Fall der Form eines Heidemann-Spatels ist die Form eines über die Kante abgewinkelten Heidemann-Spatels bevorzugt. Der Heidemann-Spatel sollte vorteilhafterweise ein schmaler Heidemann-Spatel sein, bevorzugt ein über die Kante abgewinkelter, schmaler Heidemann-Spatel. Im Fall der Form einer Sonde ist die Form einer abgerundeten Sonde bevorzugt. Der Behandler kann die geeignete Form des Positionierungselementes nach seinen Vorstellungen und Erfahrungen wählen. Je nachdem mit welchem aus dem Stand der Technik bekannten Instrument der Behandler gewohnt ist zu arbeiten, wird er beispielsweise eine Variante des erfindungsgemäßen Instrumentes wählen, dessen Positionierungselement in Form eines Heidemann-Spatels oder einer abgerundeten Sonde gestaltet ist.

Der Spiegel ist vorzugsweise ein runder Spiegel, beispielsweise ein Spiegel mit einem Durchmesser zwischen 10 und 20 mm, vorzugsweise von 14 mm. Er weist zweckmäßigerweise einen Grundkörper mit einer Rückseite und einer Vorderseite auf. Die Vorderseite des Grundkörpers kann als reflektierende Fläche ausgebildet sein. Alternativ kann auf der Vorderseite des Grundkörpers eine Beschichtung aus einem reflektierenden Material aufgebracht sein. Mit Ausnahme dieser Beschichtung kann das erfindungsmäße Instrument vollständig aus rostfreiem Edelstahl bestehen. Bei dem Grundkörper kann es sich jedoch auch um einen Glaskörper handeln. Auf die Vorderseite oder die Rückseite des Glaskörpers können reflektierende Schichten aufgebracht sein. Der Grundkörper, insbesondere der Glaskörper, kann in eine den Grundkörper haltende Fassung eingebracht sein, die eine Rückwand, an der der Grundkörper mit seiner Rückseite anliegt, und einen Rand, der an der Mantelfläche des Grundkörpers anliegt, aufweist. Ist der Grundkörper ein Glaskörper, so kann vorgesehen sein, dass das erfindungsgemäße Instrument, mit Ausnahme des Grundkörpers und, falls vorhanden, dessen Schichten, aus rostfreiem Edelstahl besteht. Der Grundkörper ist vorzugsweise zylinderförmig. In diesem Fall sollte der Rand der Fassung ringförmig sein. Der Spiegel kann den Spiegeln gleichen, die bei bekannten zahnmedizinischen Mundspiegeln eingesetzt werden.

Das erfindungsgemäße Instrument besteht zweckmäßigerweise vollständig aus einem oder mehreren desinfizierbaren und sterilisierbaren Materialien.

Ist der Spiegel abgewinkelt zur Haupterstreckungsrichtung des Griffes angeordnet, so ist es bevorzugt, dass zwischen der Vorderseite des Grundkörpers des Spiegels und der Haupterstreckungsrichtung des Griffes ein stumpfer Winkel ausgebildet ist, so dass zwischen der Rückseite des Grundkörpers des Spiegels und der Haupterstreckungsrichtung des Griffes ein spitzer Winkel ausgebildet ist.

### Erste Ausführungsform des erfindungsgemäßen Instrumentes

In einer ersten Ausführungsform der Erfindung kann vorgesehen sein, dass das Instrument einen Tragarm mit einem ersten Abschnitt aufweist, über den der Tragarm an dem Arbeitsende des Griffs lösbar oder unlösbar befestigt ist. Zur lösbaren Befestigung des Tragarms an dem Griff kann eine Schraubverbindung zwischen dem ersten Abschnitt des Tragarms und dem Griff vorgesehen sein. Beispielsweise kann an dem Arbeitsende des Griffes ein Gewinde ausgebildet sein, wobei das Gegengewinde an einer Stirnseite des ersten Abschnittes des Tragarms ausgebildet ist. Bei dem Gewinde am Arbeitsende des Griffes kann es sich um ein Innengewinde handeln, bei dem Gegengewinde am ersten Abschnitt des Tragarmes um ein Außengewinde. Das Innengewinde am Arbeitsende des Griffes kann in einer Bohrung ausgebildet sein, die sich in der Stirnseite des Griffes befindet. Alternativ kann das Außengewinde am Arbeitsende des Griffes und das Innengewinde an der Stirnseite des ersten Abschnittes des Tragarms ausgebildet sein. Bei den Gewinden kann es sich um Normgewinde handeln. Die lösbare Verbindung zwischen dem Tragarm und dem Griff ermöglicht einen Austausch des Tragarms und damit der am Tragarm befestigten Bauelemente des erfindungsgemäßen Instrumentes. Bei diesen Bauelementen kann es sich um den Spiegel, das Positionierungselement oder beides handeln.

Der Tragarm weist vorzugsweise einen zweiten Abschnitt auf, dessen Haupterstreckungsrichtung angewinkelt zur Haupterstreckungsrichtung des Griffes verläuft und an dem der Spiegel unlösbar oder lösbar befestigt ist. Ist der Tragarm lösbar, beispielsweise mittels einer Schraubverbindung, an dem Griff befestigt, so ist es bevorzugt, dass der Spiegel unlösbar mit dem Tragarm verbunden ist. Der Spiegel ist zweckmäßigerweise mit der Rückseite des Grundkörpers oder, wenn der Spiegel eine Fassung aufweist, mit der Fassung an dem zweiten Abschnitt befestigt, so dass die Vorderseite des Spiegels freiliegt. Dabei kann der Winkel α zwischen der Vorderseite des Grundkörpers des Spiegels und der Haupterstreckungsrichtung des Griffes ein stumpfer Winkel sein. Der zweite Abschnitt des Tragarms grenzt zweckmäßigerweise an den ersten Abschnitt des Tragarms an. Vorzugsweise verläuft der zweite Abschnitt abgewinkelt, besonders bevorzugt in einem stumpfen Winkel, zum ersten Abschnitt.

Es kann vorgesehen sein, dass an der Rückseite des Spiegels, d. h. der Rückseite des Grundkörpers oder, wenn eine Fassung vorgesehen ist, der Fassung, insbesondere der Rückwand der Fassung, das Positionierungselement befestigt ist. Das Positionierungselement kann beispielsweise mit dem Spiegel verlötet sein. Dabei ist es bevorzugt, wenn sich das Positionierungselement oder zumindest ein Segment des Positionierungselementes, in eine Richtung erstreckt, die dem Griff abgewandt ist. Bei einem aufrechtstehenden erfindungsgemäßen Instrument, bei dem die Haupterstreckungsrichtung des Griffes lotrecht verläuft, kann sich das Positionierungselement oder zumindest ein Segment davon in eine Richtung erstrecken, die parallel oder annährend parallel zur Haupterstreckungsrichtung des Griffes liegt. Bei dieser Lage des Instrumentes kann das Positionierungselement das höchstliegende Bauelement des erfindungsgemäßen Instrumentes bilden.

Vorzugsweise ist das Positionierungselement in einem Bereich des Spiegels an dessen Rückseite oder der Fassung befestigt, der von dem Griff am stärksten entfernt liegt. Bei einem aufrechtstehenden erfindungsgemäßen Instrument, bei dem die Haupterstreckungsrichtung des Griffes lotrecht verläuft, ist das der am höchsten liegende Bereich des Spiegels.

Es ist vorgesehen, dass das Positionierungselement ein erstes Segment und ein zweites Segment aufweist. Das erste Segment kann ein Verbindungselement sein, dass das zweite Segment mit dem Spiegel verbindet. Die Haupterstreckungsrichtung des ersten Segmentes verläuft vorzugsweise in der Haupterstreckungsrichtung des zweiten Abschnittes des Tragarms. Das zweite Segment ist vorzugsweise von dem ersten Segment abgewinkelt und erstreckt sich in eine Richtung, die der Vorderseite des Grundkörpers des Spiegels und damit dem Griff abgewandt ist. Der Winkel β zwischen dem ersten Segment und dem zweiten Segment ist besonders bevorzugt ein stumpfer Winkel. Das zweite Segment kann die Längenmesseinrichtung und die Kontakteinrichtung bilden.

Die Abschnitte des Tragarms sind zweckmäßigerweise längliche Körper. Sie können beispielsweise einen kreisförmigen, ellipsenförmigen oder viereckigen Querschnitt aufweisen. Das erste Segment des Positionierungselementes kann ein länglicher Körper, beispielsweise mit einem kreisförmigen, ellipsenförmigen oder viereckigen Querschnitt sein. Auch das zweite Segment des Positionierungselementes kann ein länglicher Körper, beispielsweise mit einem kreisförmigen, ellipsenförmigen oder viereckigen Querschnitt sein.

### Zweite Ausführungsform des erfindungsgemäßen Instrumentes

Die zweite Ausführungsform des erfindungsgemäßen Instrumentes entspricht der ersten Ausführungsform, außer dass das Positionierungselement nicht am dem Spiegel, sondern an dem Tragarm befestigt oder ausgebildet ist. Dazu weist der Tragarm einen dritten Abschnitt auf, dessen Haupterstreckungsrichtung in einem Winkel γ abgewinkelt zum zweiten Abschnitt des Tragarmes ist. Der dritte Abschnitt kann das Positionierungselement ganz oder teilweise bilden. Alternativ kann das Positionierungselement an dem dritten Abschnitt des Tragarms befestigt sein. Ebenso wie in der ersten Ausführungsform der Erfindung ist in der zweiten Ausführungsform der Erfindung das Positionierungselement oder zumindest ein Segment davon abgewinkelt zur Haupterstreckungsrichtung des Spiegels angeordnet und erstreckt sich in eine Richtung, die dem Griff abgewandt ist.

Der dritte Abschnitt grenzt zweckmäßigerweise an den zweiten Abschnitt an, so dass der zweite Abschnitt zwischen dem ersten und dem dritten Abschnitt angeordnet ist. Vorzugsweise erstreckt sich der dritte Abschnitt mit seiner Haupterstreckungsrichtung in einer Richtung, die, bezogen auf die Haupterstreckungsrichtung des zweiten Abschnittes, der Haupterstreckungsrichtung des ersten Abschnittes abgewandt ist. Der Winkel γ zwischen dem dritten Abschnitt des Tragarms und dem zweiten Abschnitt des Tragarms ist vorzugsweise ein stumpfer Winkel. Der Winkel γ kann dem Winkel β zwischen dem ersten Abschnitt des Tragarms und dem zweiten Abschnitt des Tragarms entsprechen.

Die Abschnitte des Tragarms sind zweckmäßigerweise längliche Körper. Sie können beispielsweise einen kreisförmigen, ellipsenförmigen oder viereckigen Querschnitt aufweisen.

### Dritte Ausführungsform des erfindungsgemäßen Instrumentes

In einer dritten Ausführungsform der Erfindung weist das erfindungsgemäße Instrument auf:
- einen ersten Tragarm, an dem der Spiegel lösbar oder unlösbar befestigt ist; und
- einen zweiten Tragarm an dem das Positionierungselement ausgebildet oder befestigt ist;
wobei der zweite Tragarm drehbar zum Griff ausgebildet ist. Dabei kann vorgesehen sein, dass die Drehachse des zweiten Tragarms koaxial oder achsparallel zur Hauptstreckungsrichtung des Griffes verläuft. Um eine Drehung des zweiten Tragarms zu ermöglichen, kann vorgesehen sein, dass der zweite Tragarm einen ersten und einen zweiten Abschnitt aufweist, wobei der erste Abschnitt in eine Bohrung eingeführt ist, die in dem Griff ausgebildet ist. An dem zweiten Abschnitt des zweiten Tragarms kann das Positionierungselement ausgebildet sein.

Der Griff kann ein erstes Griffteil und ein zweites Griffteil aufweisen. In dem ersten Griffteil kann eine Durchgangsbohrung ausgebildet sein, die axial zur Haupterstreckungsrichtung des Griffes verläuft. Auch im zweiten Griffteil kann eine Bohrung ausgebildet sein, die axial zur Haupterstreckungsrichtung des Griffes verläuft. Die Bohrung im zweiten Griffteil ist zumindest an einer Stirnseite des zweiten Griffteils offen. Die Bohrung im zweiten Griffteil kann eine Durchgangsbohrung sein. Der Durchmesser der Durchgangsbohrung im zweiten Griffteil sollte zumindest in dem Abschnitt, der dem ersten Griffteil zugewandt ist, dem Durchmesser der Durchgangsbohrung im ersten Griffteil entsprechen.

Das erste Griffteil und das zweite Griffteil sind miteinander durch ein Verbindungsmittel verbunden, wobei die Durchgangsbohrung des ersten Griffteils fluchtend zur Bohrung des zweiten Griffteils verläuft und die Bohrung des zweiten Griffteils zur Durchgangsbohrung hin geöffnet ist. Dabei sollten die einander zugewandten Stirnseiten der beiden Griffteil voneinander unter Ausbildung eines Spaltes beabstandet sein. Der Spalt erstreckt sich in Umfangsrichtung des Griffes. Das Verbindungmittel kann ein Steg sein, der sich von der Stirnseite des ersten Griffteils, die dem zweiten Griffteil zugewandt ist, zur gegenüberliegenden Stirnseite des zweiten Griffteils erstreckt. Das Verbindungsmittel kann fluchtend zur äußeren und/oder inneren Mantelfläche des ersten Griffteils und des zweiten Griffteils ausgebildet sein. Das Verbindungselement kann bogenförmig sein, wobei es um eine Längsachse gekrümmt ist, die koaxial zur Haupterstreckungsrichtung des Griffes verläuft. Die Ausdehnung des Spaltes in Umfangsrichtung ist dann durch das Verbindungsmittel begrenzt. Die Ausdehnung des ersten Griffteils und des zweiten Griffteils in radialer Richtung sollten einander entsprechen, so dass der Mantel des ersten Griffteils und des zweiten Griffteils die gleiche Stärke haben. Die Ausdehnung des ersten Griffteils in der Haupterstreckungsrichtung des Griffes ist vorzugsweise geringer als die des zweiten Griffteiles. Der Spalt zwischen den beiden Griffteilen liegt dann nicht in der Mitte, bezogen auf die Haupterstreckungsrichtung des Griffes, sondern zwischen der Mitte und dem Arbeitsende des Griffes.

Der erste Abschnitt des zweiten Tragarms ist durch die Durchgangsbohrung des ersten Griffteils in die Bohrung des zweiten Griffteils geführt. In Höhe des Spaltes zwischen dem ersten Griffteil und dem zweiten Griffteil kann an dem ersten Abschnitt des zweiten Tragarms ein Stellelement ausgebildet sein, das sich in radialer Richtung erstreckt. Dabei kann sich das Stellelement in radialer Richtung bis in Höhe der äußeren Mantelfläche des Griffes oder darüber hinaus erstrecken. Das Stellelement kann einen Rand aufweisen, der mit einer Riffelung oder ein anderen Oberflächengestaltung versehen ist, um die Handhabung des erfindungsgemäßen Instrumentes zu erleichtern.

Das Stellelement kann ein scheibenförmiger Körper sein, beispielsweise ein radförmiger Körper. In dem Stellelement ist ein Führungsloch ausgebildet, durch die das Verbindungsmittel des Griffes geführt ist. Das Führungsloch kann außermittig zur Längsachse des ersten Abschnittes des zweiten Griffteils angeordnet sein. Bei dem Führungsloch kann es sich um ein gekrümmtes Langloch, insbesondere ein C-förmiges Langloch handeln. Die Krümmung des gekrümmten Langloches korrespondiert mit der Krümmung des Verbindungselementes. Vorzugsweise ist das gekrümmte Langloch so groß, dass das Positionierungselement um einen Betrag gedreht werden kann, der bis zu 270° beträgt. Die Ausdehnung des Stellelementes in axialer Richtung sollte geringer als die Ausdehnung des Spaltes zwischen den beiden Griffteilen in dieser Richtung sein.

Um eine unerwünschte Drehung des zweiten Tragarms zu verhindern, kann ein Fixierungselement vorgesehen sein, mit dem der zweite Tragarm lösbar in dem Griff fixiert werden kann. Bei dem Fixierungselement kann es sich beispielsweise um ein Federelement, insbesondere um eine Druckfeder, handeln. Es kann vorgesehen sein, dass der erste Abschnitt an seinem dem zweiten Abschnitt abgewandten Ende in eine Hülse eingeführt ist. Die Hülse kann mit dem ersten Abschnitt verschraubt sein. Dazu kann die Hülse ein Innengewinde aufweisen, in das der erste Abschnitt des zweiten Tragarms mit einem Gegengewinde, das in der Mantelfläche des ersten Abschnittes ausgebildet ist, greift. Am umlaufenden Rand der Hülse können Zähne ausgebildet sein. Die Zähne der Hülse greifen unter Einwirkung des Federelementes in korrespondierende Zähne ein, die in der Bohrung des zweiten Greifteils ausgebildet sind. Die Zähne der Hülse können sich dabei in axialer Richtung und nicht in radialer Richtung erstrecken. Die maximale Ausdehnung der Zähne der Hülse in axialer Richtung sollte geringer als die Differenz zwischen der Ausdehnung des Spaltes und der Ausdehnung des Stellelementes, jeweils in axialer Richtung, sein. Das Federelement presst die Hülse gegen die korrespondierenden Zähne in der inneren Mantelfläche des zweiten Griffteils. Gleichzeitig presst das Federelement das in dem Spalt befindliche Stellelement gegen die Stirnseite des ersten Griffteiles. Damit ist der zweite Tragarm in dem Griff fixiert. Drückt nun der Behandler das Stellelement von der Stirnseite des ersten Griffteils zur Stirnseite des zweiten Griffteiles werden die Zähne der Hülse aus den korrespondierenden Zähnen im zweiten Griffteil gelöst, so dass der zweite Tragarm gedreht werden kann. Sobald der Behandler das Ausüben von Druck auf das Stellelement aufgibt, presst das Federelement das Stellelement wieder gegen die Stirnseite des ersten Griffteiles und die Zähne der Hülse in die korrespondierenden Zähne in der inneren Mantelfläche des zweiten Griffteils. Damit ist der zweite Tragarm wieder in dem Griff fixiert. Die Zähne der Hülse können einen Zahnkranz bilden. Die korrespondierenden Zähne in der inneren Mantelfläche des zweiten Griffteils bilden dann einen korrespondierenden Zahnkranz.

Die Drehung des zweiten Tragarmes um die Haupterstreckungsrichtung seines ersten Abschnittes kann auf vorgegebene Winkelpositionen beschränkt sein. Beispielsweise kann vorgesehen sein, dass nur eine Drehung möglich ist, die einem vorgegebenen Winkelabstand entspricht. Beispielsweise kann ein Winkelabstand von 90°, 180° oder 270° vorgegeben sein. Damit können Drehungen von einer Ausgangsposition, der Nullposition, um bis zu 270° bewirkt werden. Um eine Drehung auf vorgegebene Winkelpositionen zu beschränken, ist eine entsprechende Anzahl und Anordnung der Zähne der Hülse und der korrespondierenden Zähnen in der inneren Mantelfläche des zweiten Griffteils erforderlich.

Ist die Bohrung in dem zweiten Griffteil als Durchgangsbohrung ausgebildet, so kann vorgesehen sein, dass die Stirnseite des zweiten Griffteiles, die dem ersten Griffteil abgewandt ist, mit einer Kappe verschlossen ist, beispielsweise über eine Schraubverbindung. Dazu kann in der inneren Mantelfläche des zweiten Griffteils ein Innengewinde ausgebildet sein, in das ein von der Kappe abstehender Stift, der ein Gegengewinde an seiner Außenseite aufweist, greift. Zwischen der Kappe und dem Ende des ersten Abschnittes des zweiten Griffteils, das der Kappe zugewandt ist, ist das Federelement angeordnet.

Der erste Griffteil, der zweite Griffteil und das Verbindungsmittel können einstückig ausgebildet sein. Es kann alternativ vorgesehen sein, dass das erste Griffteil und das Verbindungsmittel einstückig ausgebildet sind, wobei das Verbindungsmittel an dem zweiten Griffteil befestigt ist, beispielsweise durch eine Steck- und/oder Schraubverbindung. Der erste Tragarm kann einstückig mit dem Griff oder, wenn der Griff nicht einstückig ist, mit dem ersten Griffteil ausgebildet sein.

Es kann vorgesehen sein, dass die Haupterstreckungsrichtung des zweiten Abschnittes des zweiten Tragarms zur Hauptstreckungsrichtung des Griffes um einen Winkel ε abgewinkelt ist.

Es kann vorgesehen sein, dass der erste Tragarm einen ersten Abschnitt, über den der erste Tragarm an dem Arbeitsende des Griffs befestigt ist, und einen zweiten Abschnitt aufweist, dessen Haupterstreckungsrichtung zur Hauptstreckungsrichtung des Griffes um einen Winkel δ abgewinkelt ist und an dem der Spiegel lösbar oder unlösbar befestigt ist. Der erste Abschnitt des ersten Tragarms kann eine Bohrung zur Lagerung des zweiten Tragarms in dem ersten Tragarm aufweisen. In diesem Fall kann der erste Abschnitt des zweiten Tragarms durch den ersten Abschnitt des ersten Tragarms geführt werden, so dass eine Drehung des zweiten Tragarmes im ersten Abschnitt des ersten Tragarmes möglich ist. Die Bohrung im ersten Abschnitt des ersten Tragarms kann fluchtend zur Durchgangsbohrung des ersten Griffteils ausgebildet sein. Der Durchmesser der Bohrung kann dem Durchmesser der Durchgangsbohrung entsprechen.

Der Winkel δ ist vorzugsweise ein stumpfer Winkel. Der Winkel ε ist vorzugsweise ein stumpfer Winkel. Der zweite Abschnitt des ersten Tragarms grenzt vorzugsweise an den ersten Abschnitt des ersten Tragarms an. Der zweite Abschnitt des zweiten Tragarms grenzt vorzugsweise an den ersten Abschnitt des zweiten Tragarms an.

Der zweite Abschnitt des zweiten Tragarms kann das Positionierungselement ganz oder teilweise bilden. Alternativ kann das Positionierungselement an dem zweiten Abschnitt des zweiten Tragarms befestigt sein.

Die Abschnitte der beiden Tragarme sind zweckmäßigerweise längliche Körper. Sie können beispielsweise einen kreisförmigen, ellipsenförmigen oder viereckigen Querschnitt aufweisen.

### Vierte Ausführungsform des erfindungsgemäßen Instrumentes

In einer vierten Ausführungsform der Erfindung weist das erfindungsgemäße Instrument auf:
- einen ersten Tragarm mit einem ersten Abschnitt, über den der erste Tragarm an dem Arbeitsende des Griffs befestigt ist, wobei der erste Tragarm einen zweiten Abschnitt aufweist, dessen Haupterstreckungsrichtung zur Hauptstreckungsrichtung des Griffes um einen Winkel δ abgewinkelt ist, und an dem der Spiegel lösbar oder unlösbar befestigt ist; und
- einen zweiten Tragarm mit einem ersten Abschnitt, über den der zweite Tragarm an dem Arbeitsende des Griffs oder an dem ersten Tragarm befestigt ist, wobei der zweite Tragarm einen zweiten Abschnitt aufweist, dessen Haupterstreckungsrichtung zur Hauptstreckungsrichtung des Griffes um einen Winkel ε abgewinkelt ist.

Der Winkel δ ist vorzugsweise ein stumpfer Winkel. Der Winkel ε ist vorzugsweise ein stumpfer Winkel. Der zweite Abschnitt des ersten Tragarms grenzt vorzugsweise an den ersten Abschnitt des ersten Tragarms an. Der zweite Abschnitt des zweiten Tragarms grenzt vorzugsweise an den ersten Abschnitt des zweiten Tragarms an.

Der zweite Abschnitt des zweiten Tragarms kann das Positionierungselement ganz oder teilweise bilden. Alternativ kann das Positionierungselement an dem zweiten Abschnitt des zweiten Tragarms befestigt sein.

Es kann vorgesehen sein, dass der zweite Tragarm um eine Drehachse drehbar an dem Arbeitsende des Griffs oder an dem ersten Tragarm befestigt ist, wobei die Drehachse koaxial oder achsparallel zur Hauptstreckungsrichtung des Griffes verläuft. Um eine Drehung des zweiten Tragarms zu ermöglichen, kann vorgesehen sein, dass der erste Abschnitt des ersten Tragarms eine Bohrung zur Lagerung des zweiten Tragarms in dem ersten Tragarm aufweist. In diesem Fall ist der erste Abschnitt des zweiten Tragarms in dem ersten Abschnitt des ersten Tragarms drehbar gelagert. Zu diesem Zweck kann der erste Abschnitt des ersten Tragarms ein an seinen beiden Stirnseiten offener Hohlzylinder sein, wobei der erste Abschnitt des zweiten Tragarmes durch den ersten Abschnitt des ersten Tragarmes geführt ist. Der erste Abschnitt des zweiten Tragarmes kann innerhalb des Griffes mit einem Überstand aus dem ersten Abschnitt des ersten Tragarmes herausstehen. In den Überstand kann ein Stellelement eingreifen, das eine Drehung des zweiten Tragarmes ermöglichen kann. Der Überstand kann an seiner dem ersten Tragarm abgewandten Stirnseite eine Bohrung aufweisen, in die ein Fixierungselement zur lösbaren Fixierung des zweiten Tragarmes in den Griff eingreift. Zur Drehung des ersten Abschnittes des zweiten Tragarms kann dann mittels des Stellelements die Fixierung des zweiten Tragarmes in dem Griff gelöst und wieder hergestellt werden.

Die Drehung des zweiten Tragarmes um die Haupterstreckungsrichtung seines ersten Abschnittes kann auf vorgegebene Winkelpositionen beschränkt sein. Beispielsweise kann vorgesehen sein, dass nur eine Drehung möglich ist, die einem vorgegebenen Winkelabstand entspricht. Beispielsweise kann ein Winkelabstand von 90°, 180° oder 270° vorgegeben sein. Damit können Drehungen von einer Ausgangsposition, der Nullposition, um bis zu 270° bewirkt werden. Um eine Drehung auf vorgegebene Winkelpositionen zu beschränken, kann eine schlitzartige Öffnung in dem Griff, die sich in Umfangsrichtung des Griffes erstreckt, vorgesehen sein, die die Drehung ermöglicht, aber auch begrenzt. Durch die Öffnung ist das Stellelement, beispielsweise ein Stift, geführt. Das Stellelement kann eine Führungshülse für den Stift aufweisen, die an dem Überstand des ersten Abschnittes des zweiten Tragarms befestigt ist, an der Außenseite des Griffes übersteht und innerhalb der schlitzartigen Öffnung bewegt werden kann.

Weist der Griff an seinem Arbeitsende eine Bohrung auf, in die der erste Abschnitt des ersten Tragarms eingeführt ist und in die sich der Überstand des ersten Abschnitts des zweiten Tragarms erstreckt, so können in der Bohrung Rastmittel in Form von Rastkerben für das Fixierungselement ausgebildet sein. In die Rastkerben rastet das Fixierungsmittel mit seinem freiliegenden Ende ein, sofern die Fixierung des zweiten Tragarmes in dem Griff nicht mittels des Stellelementes gelöst ist.

Die Abschnitte der beiden Tragarme sind zweckmäßigerweise längliche Körper. Sie können beispielsweise einen kreisförmigen, ellipsenförmigen oder viereckigen Querschnitt aufweisen.

### Verwendung des erfindungsgemäßen Instrumentes

Das erfindungsgemäße Instrument, einschließlich der vorstehend beschrieben ersten bis vierten Ausführungsform, vereinfacht und beschleunigt die Positionierung von Brackets auf Zahnoberflächen. Der Wechsel zwischen dem Spiegel und dem Positionierelement der erfindungsgemäßen Vorrichtung nimmt weniger Zeit in Anspruch, da auf das mehrfache separate Anreichen der Instrumente durch die Zahnarzthelferin verzichtet werden kann. Gleichzeitig sinkt die Fehleranfälligkeit. Bei der Positionierung eines Brackets auf einer Zahnoberfläche kann in vier Arbeitsschritten vorgegangen werden, ohne dass der Behandler das erfindungsgemäße Instrument aus der Hand legen muss. Nach dem Aufbringen des Brackets auf den Zahn kann zur Positionierung des Brackets folgendermaßen vorgegangen werden:
(i) Korrektur der Neigung bzw. Angulation des Brackets in Bezug auf die Längsachse des Zahnes mittels des Positionierungselementes, insbesondere der Kontakteinrichtung, und Entfernung des Adhäsiv-Überschusses mittels des Positionierungselementes, insbesondere der Kontakteinrichtung.
(ii) Überprüfen der Brackethöhe, d. h. der vertikalen Positionierung, unter Verwendung des Positionierungselementes, insbesondere der Längenmesseinrichtung, beispielsweise der Skala, und Korrektur der Brackethöhe, wenn die Überprüfung ergibt, dass die tatsächliche Brackethöhe nicht der erwünschten Brackethöhe entspricht mittels des Positionierungselementes, insbesondere der Kontakteinrichtung. Die Korrektur der Brackethöhe kann auch als "vertikale Korrektur" bezeichnet werden.
(iii) Überprüfen der mesio-distalen Position des Brackets, d. h. der horizontalen Positionierung, mittels des Spiegels und Korrektur der mesio-distalen Position des Brackets mittels des Positionierungselementes, insbesondere der Kontakteinrichtung. Die Korrektur der mesio-distalen Position des Brackets kann auch als "horizontale Korrektur" bezeichnet werden.

Das erfindungsgemäß vorgesehene Positionierungselement kann zur Bestimmung der Position des Brackets am Zahn und zur Positionierung des Brackets am Zahn verwendet werden. Insbesondere kann das Positionierungselement zur Bestimmung der vertikalen Position des Brackets am Zahn und zur Positionierung des Brackets am Zahn verwendet werden. Der erfindungsgemäß vorgesehene Spiegel kann ebenfalls zur Bestimmung der Position des Brackets am Zahn verwendet werden. Insbesondere kann der Spiegel zur Bestimmung der horizontalen Position des Brackets am Zahn verwendet werden.

Die Erfindung wird nachstehend anhand von Ausführungsbeispielen, die die Erfindung nicht einschränken sollen, unter Bezugnahme auf die Zeichnungen näher erläutert. Dabei zeigen
- Fig. 1: eine perspektive Seitendarstellung einer ersten Ausführungsform des erfindungsgemäßen Instrumentes;
- Fig. 2: eine perspektive Darstellung der ersten Ausführungsform des erfindungsgemäßen Instrumentes von vorn;
- Fig. 3: eine Teildarstellung der ersten Ausführungsform des erfindungsgemäßen Instrumentes;
- Fig. 4: eine Teildarstellung einer zweiten Ausführungsform des erfindungsgemäßen Instrumentes;
- Fig. 5: eine Teilschnittdarstellung einer dritten Ausführungsform des erfindungsgemäßen Instrumentes;
- Fig. 6a: eine Darstellung eines Schnittes quer durch den ersten Abschnitt des ersten Tragarms der dritten Ausführungsform des erfindungsgemäßen Instrumentes;
- Fig. 6b: eine Darstellung eines Ausschnittes des Griffes im Bereich des Stellelementes der dritten Ausführungsform des erfindungsgemäßen Instrumentes im Längsschnitt;
- Fig. 6c: eine Darstellung der dritten Ausführungsform des erfindungsgemäßen Instrumentes im Querschnitt (Schnittlinie A--A in Fig. 6b);
- Fig. 6d: eine perspektivische Darstellung einer Hülse der dritten Ausführungsform des erfindungsgemäßen Instrumentes;
- Fig. 6e: eine Darstellung von Bestandteilen der dritten Ausführungsform des erfindungsgemäßen Instrumentes;
- Fig. 7: eine Teilschnittdarstellung einer vierten Ausführungsform des erfindungsgemäßen Instrumentes;
- Fig. 8a: eine Darstellung eines Teils der vierten Ausführungsform des erfindungsgemäßen Instrumentes im Längsschnitt;
- Fig. 8b: eine Darstellung der vierten Ausführungsform des erfindungsgemäßen Instrumentes im Querschnitt (Schnittlinie A--A in Fig. 8a);
- Fig. 8c: eine vergrößerte Darstellung des Ausschnittes B von Fig. 8a;
- Fig. 8d: eine Seitenansicht eines Teil der vierten Ausführungsform des erfindungsgemäßen Instrumentes im Bereich des Stellelementes;
- Fig. 8e: eine Draufsicht auf die vierte Ausführungsform des erfindungsgemäßen Instrumentes in Ausgangsposition;
- Fig. 8f: eine Draufsicht auf die vierte Ausführungsform des erfindungsgemäßen Instrumentes mit zur Ausgangsposition verstelltem zweiten Tragarm; und
- Fig. 9: eine schematische Darstellung von Schneidezähnen eines Unterkiefers zur Veranschaulichung der vertikalen und horizontalen Positionierung eines Brackets auf einem Zahn.

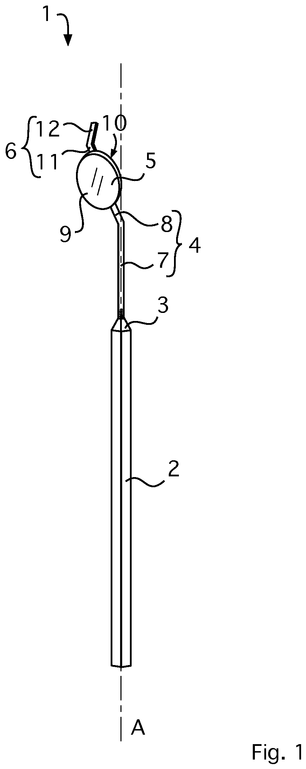

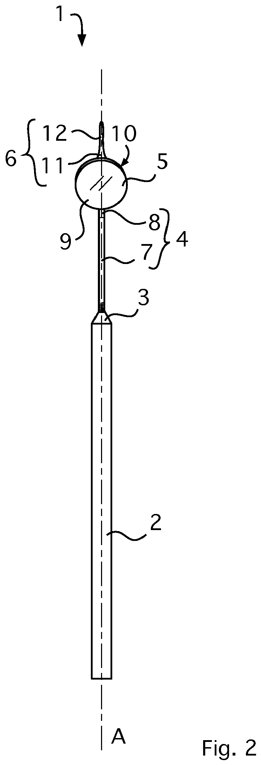

Die in den Figuren 1 bis 3 gezeigte erste Ausführungsform des erfindungsgemäßen Instrumentes 1 weist einen Griff 2 auf, an dessen Arbeitsende 3 ein Tragarm 4 für einen Spiegel 5 lösbar befestigt ist. An dem Spiegel 5 ist ein Positionierungselement 6 angebracht. Der Spiegel 5 und das Positionierungselement 6 sind am selben Ende des Griffes 2, dem Arbeitsende 3, angeordnet.

Der Griff 2 ist ein stielförmiger Körper, dessen Haupterstreckungsrichtung seine Längsachse A ist. An einer Stirnseite des Griffes 2, dem Arbeitsende 3, ist der Tragarm 4 über eine Schraubverbindung an dem Griff 2 befestigt. Die Schraubverbindung ermöglicht das gemeinsame Austauschen des Tragarms 4, des am Tragarm 4 befestigten Spiegels 5 und des am Spiegel befestigten Positionierungselementes 6.

Der Tragarm 4 weist einen ersten Abschnitt 7 und einen an den ersten Abschnitt 7 angrenzenden zweiten Abschnitt 8 auf. Der erste Abschnitt 7 des Tragarms 4 ist ein länglicher Körper mit einem annähernd kreisförmigen Querschnitt. An einer Stirnseite weist der erste Abschnitt 7 ein Gewinde (nicht gezeigt) auf, mit dem er mit dem Griff 2 an dessen Arbeitsende 3 verschraubt ist. An die andere Stirnseite des ersten Abschnittes 7 grenzt der zweite Abschnitt 8 des Tragarms 4 mit einer Stirnseite an. Der zweite Abschnitt 8 kann ebenfalls ein länglicher Körper mit einem annähernd kreisförmigen Querschnitt sein. Der zweite Abschnitt 8 ist in der Regel kürzer als der erste Abschnitt 7.

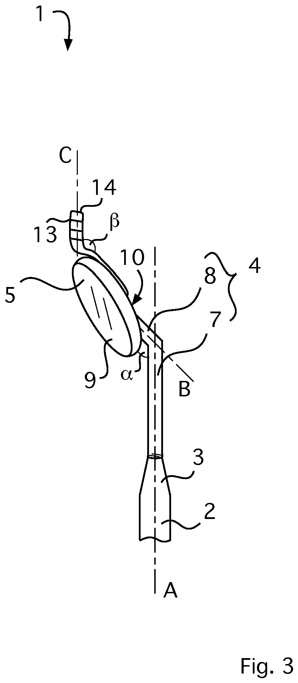

Es ist in den Figuren 1 bis 3 zu erkennen, dass die Haupterstreckungsrichtung des ersten Abschnittes 7, seine Längsachse, auf der Längsachse A des Griffes 2 liegt. Der zweite Abschnitt 8 ist zum ersten Abschnitt 7 abgewinkelt. Die Haupterstreckungsrichtung des zweiten Abschnittes 8, dessen Längsachse, verläuft in einem stumpfen Winkel α zur Längsachse A des Griffes 2 und zur Längsachse des ersten Abschnittes 7 (siehe Fig. 3). An dem zweiten Abschnitt 8 ist der Spiegel 5 mit seiner Rückseite 10 befestigt. Dabei kann es sich um die Rückwand einer Fassung (nicht gezeigt) oder die Rückseite eines Grundkörpers handeln. Die Vorderseite 9 des Spiegels 5 ist eine reflektierende Oberfläche und damit die eigentliche Spiegelfläche. Die Vorderseite 9 des Spiegels 5 ist dem Griff 2, insbesondere dessen Arbeitsende zugewandt, die Rückseite 10 ist dem Griff 2 abgewandt. Die erste Ausführungsform des erfindungsgemäßen Instrumentes 1 ist in den Figuren 1 bis 3 als aufrechtstehendes Instrument gezeigt, bei dem die Haupterstreckungsrichtung des Griffes 2, seine Längsachse A, lotrecht verläuft. In dieser Lage des Instrumentes 1 weist die Vorderseite 9 des Spiegels 5 nach unten, die Rückseite 10 hingegen nach oben.

An der Rückseite 10 des Spiegels 5 ist das Positionierungselement 6 befestigt. Das Positionierungselement ist dabei in einem Bereich an der Rückseite befestigt, der, bezogen auf die Rückseite 10 des Spiegels 5, der Verbindungsstelle zwischen dem zweiten Abschnitt 8 und der Rückseite 10 des Spiegels 5 gegenüberliegt. Bei einem aufrechtstehenden Instrument ist somit das Positionierungselement 6 im höchstliegenden Bereich der Rückseite 10 des Spiegels 5 befestigt.

Das Positionierungselement 6 weist in der gezeigten Ausführungsform ein erstes Segment 11 und ein zweites Segment 12 auf. Das erste Segment 11 ist ein länglicher Körper, dessen Haupterstreckungsrichtung annährend auf der Längsachse B des zweiten Abschnittes 8 des Tragarms 4 liegt oder annähernd parallel dazu verläuft. Das erste Segment 11 erstreckt sich dabei in eine Richtung, die dem Griff 2 und dem zweiten Abschnitt 8 des Tragarms 4 abgewandt ist. An das erste Segment 11 grenzt das zweites Segment 12 an, dass zum ersten Segment 11 abgewinkelt ist, und zwar in einem stumpfen Winkel β. Das zweite Segment 12 erstreckt sich dabei in entgegengesetzter Richtung zum ersten Abschnitt 7 des Tragarms 4. Das zweite Segment 12 kann ein länglicher Körper sein, dessen Längsseite um 90° zur Längsseite von Segment 11 gedreht ist. Die Haupterstreckungsrichtung (Längsachse C in Fig. 3) des zweiten Segmentes 12 kann annährend parallel zur Längsachse A des Griffes 2 verlaufen. Das zweite Segment 12 weist die Längenmesseinrichtung 13 des Positionierungselementes 6, eine Skala, auf. Je nach geometrischer Form des zweiten Segmentes bilden dessen Oberflächen, beispielsweise dessen Flächenseiten, Kanten und/oder Stirnseiten, die Kontakteinrichtung 14 des Positionierungselementes 6. Ist das zweite Segment 12 ein flacher Körper, so bilden die Kanten des zweiten Segmentes 12 und seine Flächenseiten die Kontakteinrichtung 14. Das ist bei dem Werkzeug eines Heidemann-Spatels der Fall. Ist das zweite Segment 12 ein stiel- oder stangenförmiger Körper, so bilden die Mantelfläche des zweiten Segmentes 12 und seine Spitze oder abgerundete oder nicht-abgerundete Stirnseite die Kontakteinrichtung 14. Das ist bei dem Werkzeug einer Sonde der Fall.

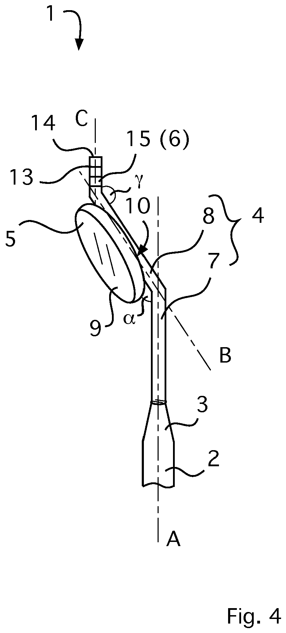

Die in Fig. 4 gezeigte zweite Ausführungsform des erfindungsgemäßen Instrumentes 1 entspricht der ersten Ausführungsform, außer dass der Tragarm 4 einen dritten Abschnitt 15 aufweist, der mit einer seiner Stirnseiten an eine Stirnseite des zweiten Abschnittes 8 angrenzt, so dass der zweite Abschnitt 8 zwischen dem ersten Abschnitt 7 und dem dritten Abschnitt 15 angeordnet ist, und das Positionierungselement 6 nicht am dem Spiegel 5, sondern an dem Tragarm 4 ausgebildet ist.

Der dritte Abschnitt 15 ist mit seiner Haupterstreckungsrichtung (Längsachse C in Fig. 4) in einem stumpfen Winkel γ abgewinkelt zur Haupterstreckungsrichtung des zweiten Abschnittes 8 des Tragarmes 4 angeordnet. Der dritte Abschnitt 15 kann ein länglicher Körper sein. Die Haupterstreckungsrichtung des dritten Abschnittes 15 kann annähernd parallel zur Längsachse A des Griffes 2 verlaufen. Der dritte Abschnitt 15 weist die Längenmesseinrichtung 13 des Positionierungselementes 6, eine Skala, auf. Je nach geometrischer Form des dritten Abschnittes 15 bilden dessen Oberflächen, beispielsweise dessen Flächenseiten, Kanten und/oder Stirnseiten, die Kontakteinrichtung 14 des Positionierungselementes 6. Ist der dritte Abschnitt 15 ein flacher Körper, so bilden die Kanten des dritten Abschnittes 15 und seine Flächenseiten die Kontakteinrichtung 14. Das ist bei dem Werkzeug eines Heidemann-Spatels der Fall. Ist der dritte Abschnitt 15 ein stiel- oder stangenförmiger Körper, so bilden die Mantelfläche des dritten Abschnittes 15 und seine Spitze oder abgerundete oder nicht-abgerundete Stirnseite die Kontakteinrichtung 14. Das ist bei dem Werkzeug einer Sonde der Fall.

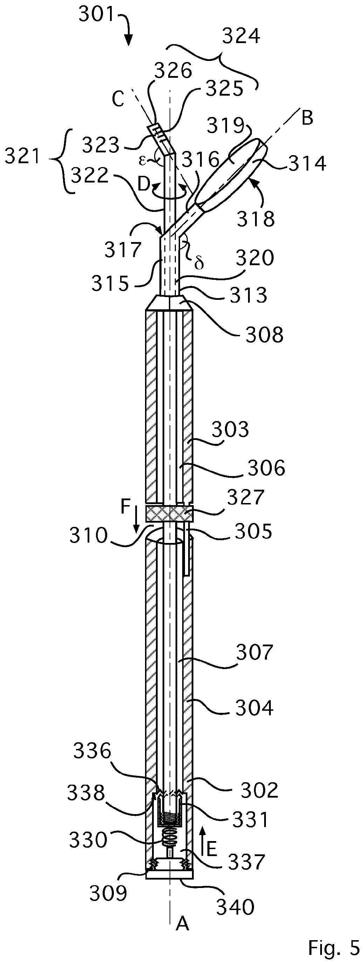

Die in den Figuren 5 bis 6e gezeigte dritte Ausführungsform des erfindungsgemäßen Instrumentes 301 weist einen Griff 302 mit einem ersten Griffteil 303 und einem zweiten Griffteil 304 auf. Das erste Griffteil 303 weist eine Durchgangsbohrung 306 auf, die fluchtend zu einer Durchgangsbohrung 307 in dem zweiten Griffteil 304 ausgebildet ist. An der Stirnseite des ersten Griffteiles 303, die dem zweiten Griffteil 304 abgewandt ist, befindet sich das Arbeitsende 308. An seiner anderen Stirnseite weist das Griffteil 303 einen als Verbindungsmittel dienenden Steg 305 auf, der sich in Richtung der gegenüberliegenden Stirnseite des zweiten Griffteils 304 erstreckt. Über den Steg 305 sind das erste und das zweite Griffteil 303, 304 unter Ausbildung des Griffes 302 miteinander verbunden. Dabei ist zwischen den sich gegenüberliegenden Stirnseiten des ersten und zweiten Griffteils 303, 304 ein Spalt 310 ausgebildet. Zur Herstellung der Verbindung zwischen dem ersten Griffteil 303 und dem zweiten Griffteil 304 ist der Steg 305 in eine Ausnehmung 311 eingeführt, die im Mantel des zweiten Griffteils 304 an seiner Stirnseite ausgebildet ist. In der Ausnehmung ist der Steg 305 mittels eines Sicherungsmittels, beispielsweise einer Schraube 312, fixiert. Die Stirnseite 309 des zweiten Griffteils 304, die dem ersten Griffteil 303 abgewandt ist, bildet das andere Ende des Griffes 302.

Die Haupterstreckungsrichtung des Griffes 302 ist seine Längsachse A. Die Durchgangsbohrungen 306, 307 des ersten und zweiten Griffteils 303, 304 verlaufen koaxial zur Längsachse A des Griffes 302. Der Steg 305 ist um eine Längsachse gekrümmt, die koaxial zur Achse A des Griffes 302 verläuft. Die Ausdehnung des Spaltes 310 in Umfangsrichtung ist durch den Steg 305 begrenzt. Die Ausdehnung des ersten Griffteils 303 und des zweiten Griffteils 304 in radialer Richtung entsprechen einander, so dass der Mantel des ersten Griffteils 303 und des zweiten Griffteils 304 die gleiche Stärke haben. Die Ausdehnung des ersten Griffteils 303 in der Haupterstreckungsrichtung des Griffes 302 ist geringer als die des zweiten Griffteiles 304 in dieser Richtung. Der Spalt 310 zwischen den beiden Griffteilen 303, 304 ist somit nicht mittig ausgebildet, bezogen auf die Längsachse A des Griffes 302, und befindet sich näher am Arbeitsende 308 des Griffes 302 als an der Stirnseite 309.

Das Instrument 301 weist ferner einen ersten Tragarm 313 auf, der unlösbar mit dem Griff 302 verbunden und an dem der Spiegel 314 befestigt ist. Der erste Tragarm ist nicht drehbar, sondern starr und unlösbar an dem Arbeitsende 308 des Griffes 302 befestigt. Der erste Tragarm 313 weist einen ersten Abschnitt 315 und einen an den ersten Abschnitt 315 angrenzenden zweiten Abschnitt 316 auf. Der erste Abschnitt 315 des ersten Tragarms 313 ist ein länglicher Körper mit einem annähernd kreisförmigen Querschnitt. Die Haupterstreckungsrichtung des ersten Abschnittes 315, seine Längsachse, liegt dabei auf der Längsachse A des Griffes 302. Der erste Abschnitt 315 steht von dem Arbeitsende 308 des Griffes 302 ab, so dass der erste Abschnitt 315 mit einer ersten Stirnseite 317 freiliegt.

Der zweite Abschnitt 316 des ersten Tragarms 313 grenzt mit einer seiner Stirnseiten an die erste Stirnseite 317 des ersten Abschnittes 315 des ersten Tragarms 313 an. Er weist einen geringeren Querschnitt als der erste Abschnitt 315 auf. Der zweite Abschnitt 316 ist zum ersten Abschnitt 315 abgewinkelt. Die Haupterstreckungsrichtung des zweiten Abschnittes 316, seine Längsachse, verläuft in einem stumpfen Winkel δ zur Längsachse A des Griffes 302 und damit zur Längsachse des ersten Abschnittes 315 (siehe Längsachse B in Fig. 5). An dem zweiten Abschnitt 316 ist der Spiegel 314 lösbar befestigt. Die Vorderseite 318 des Spiegels 314 ist eine reflektierende Oberfläche und damit die eigentliche Spiegelfläche. Die Vorderseite 318 des Spiegels 314 ist dem Griff 302, insbesondere dessen Arbeitsende 308 zugewandt, seine Rückseite 319 ist dem Griff 302 abgewandt. Die dritte Ausführungsform des erfindungsgemäßen Instrumentes 301 ist Fig. 5 als aufrechtstehendes Instrument gezeigt, bei dem die Haupterstreckungsrichtung des Griffes 302, seine Längsachse A, lotrecht verläuft. In dieser Lage des Instrumentes 301 weist die Vorderseite 318 des Spiegels 314 nach unten, die Rückseite 319 hingegen nach oben. Zur lösbaren Befestigung des Spiegels 314 an dem zweiten Abschnitt 316 kann eine Schraubverbindung (nicht gezeigt) vorgesehen sein, mit der Spiegel 314 direkt auf den zweiten Abschnitt 316 aufgeschraubt werden kann. Er ist damit austauschbar. Alternativ kann zur lösbaren Befestigung des Spiegels 314 an dem zweiten Abschnitt 316 eine starre Metalllasche (nicht gezeigt) vorgesehen sein, mit der Spiegel 314 direkt auf den zweiten Abschnitt 316 aufgeschoben werden kann.

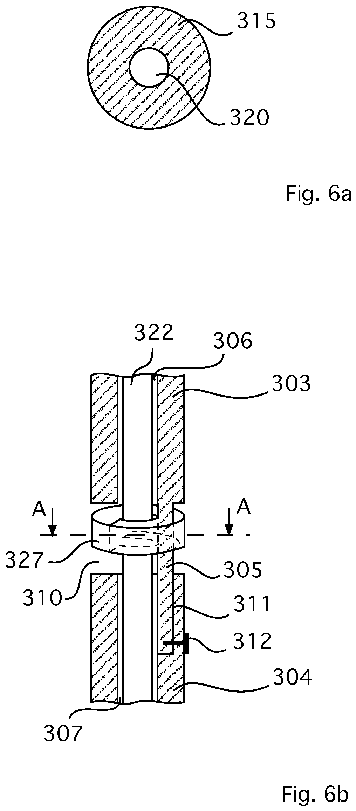

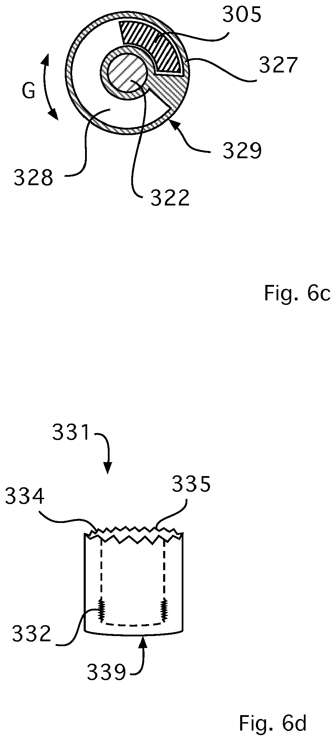

Durch den ersten Abschnitt 315 des Tragarms 313 verläuft eine zylinderförmige Durchgangsbohrung 320 (siehe Fig. 6a), die an ihren beiden Stirnseiten offen ist. Die Längsachse der Durchgangsbohrung 320 liegt koaxial zur Längsachse des ersten Abschnittes 315 des Tragarms 313 und damit zur Längsachse A des Griffes 302. Die Durchgangsbohrung ist damit fluchtend zu den Durchgangsbohrungen 306, 307 im ersten und zweiten Griffteil 303, 304 ausgebildet.

Das Instrument 301 weist ferner einen zweiten Tragarm 321 mit einem ersten Abschnitt 322 und einen an den ersten Abschnitt 322 angrenzenden zweiten Abschnitt 323 auf. Der erste Abschnitt 322 des zweiten Tragarms 321 ist ein länglicher Körper mit einem annähernd kreisförmigen Querschnitt. Der erste Abschnitt 322 ist durch die Durchgangsbohrung 320 des ersten Tragarms 313 und die Durchgangsbohrung 306 des ersten Griffteils 303 in die Durchgangsöffnung 307 des zweiten Griffteils 304 geführt. Die Haupterstreckungsrichtung des ersten Abschnittes 322, seine Längsachse, liegt dabei auf der Längsachse A des Griffes 302. Der erste Abschnitt 322 erstreckt sich über beide Stirnseiten des ersten Abschnittes 315 des ersten Tragarms 313 hinaus. Damit liegt der erste Abschnitt 322 des zweiten Tragarms 321 an der Stirnseite 317 des ersten Abschnittes 315 des ersten Tragarms 313 frei. Dabei ist die Ausdehnung des ersten Abschnittes 322 an der ersten Stirnseite 317 des ersten Tragarms 313 in Richtung der Längsachse A größer als die Ausdehnung des zweiten Abschnittes 316 des ersten Tragarms 313 und des Spiegels 314 in dieser Richtung, so dass eine Drehung des zweiten Tragarmes 321 nicht von dem ersten Tragarm 313 oder dem Spiegel 314 behindert wird.

Der zweite Abschnitt 323 des zweiten Tragarms 321 ist mit seiner Haupterstreckungsrichtung (Längsachse C in Fig. 5) in einem stumpfen Winkel ε abgewinkelt zur Hauptstreckungsrichtung, dessen Längsachse, des ersten Abschnittes 322 des zweiten Tragarmes 321 ausgebildet. Der zweite Abschnitt 323 kann ein länglicher Körper sein. Der zweite Abschnitt 323 bildet das Positionierungelement 324. Er weist dazu die Längenmesseinrichtung 325 des Positionierungselementes 324, eine Skala, auf. Je nach geometrischer Form des zweiten Abschnittes 323 bilden dessen Oberflächen, beispielsweise dessen Flächenseiten, Kanten und/oder Stirnseiten, die Kontakteinrichtung 326 des Positionierungselementes 324. Ist der zweite Abschnitt 323 ein flacher Körper, so bilden die Kanten des zweiten Abschnittes 323 und seine Flächenseiten die Kontakteinrichtung 326. Das ist bei dem Werkzeug eines Heidemann-Spatels der Fall. Ist der zweite Abschnitt 323 ein stiel- oder stangenförmiger Körper, so bilden die Mantelfläche des zweiten Abschnittes 323 und seine Spitze oder abgerundete oder nicht-abgerundete Stirnseite die Kontakteinrichtung 326. Das ist bei dem Werkzeug einer Sonde der Fall.

Der zweite Tragarm 321 ist mit seinem ersten Abschnitt 322 um die Längsachse A des Griffes 302 drehbar (Pfeil D in Fig. 5) in der die Durchgangsbohrung 320 des ersten Tragarms 313, der Durchgangsbohrung 306 des ersten Griffteils 303 und der Durchgangsöffnung 307 des zweiten Griffteils 304 gelagert. Damit der Behandler eine Drehung des zweiten Tragarms 321 bewirken kann, ist an dem ersten Abschnitt ein radförmiges Stellelement 327 ausgebildet, das sich in radialer Richtung in den Spalt 310 erstreckt. Der äußere Rand 329 des Stellelementes 327 liegt fluchtend zu den äußeren Mantelflächen des ersten und zweiten Griffteils 303, 304. Der äußere Rand 329 ist mit einer Riffelung versehen ist, um die Drehung des zweiten Tragarmes 321 zu erleichtern.

In dem Stellelement 327 ist ein Führungsloch 328 ausgebildet, durch das der Steg 305 des Griffes geführt ist. Das Führungsloch 328 ist in radialer Richtung außermittig zur Längsachse A des Griffes 302 angeordnet. Bei dem Führungsloch 328 handelt es sich um ein C-förmiges Langloch, dessen Krümmungsmittelpunkt auf der Längsachse A liegt. Der Radius des inneren Randes des Führungsloches 328 ist dabei größer als der Radius des ersten Abschnittes 322, der Radius des äußeren Randes des Führungsloches 328 ist dabei kleiner als der Radius des äußeren Randes 329 des Stellelementes 327. Das Führungsloch 328 ist in Umfangsrichtung so bemessen, dass der maximale Verdrehungsweg des Stellelementes in Bezug auf den Steg 305 eine Drehung des Positionierungselementes 324 um einen Betrag ermöglicht, der maximal 270° beträgt. Die Ausdehnung des Stellelementes 327 in axialer Richtung ist geringer als die Ausdehnung des Spaltes 310 zwischen den beiden Griffteilen 303, 304 in dieser Richtung.

Um eine unerwünschte Drehung des zweiten Tragarms 321 zu verhindern, ist eine als Fixierungselement dienende Feder 330 vorgesehen sein, mit der zweite Tragarm 321 lösbar in dem Griff 302 fixiert werden kann. Die Feder 321 ist in der Durchgangsbohrung 307 des zweiten Griffteils 304 angeordnet und presst das Stellelement 327 gegen das erste Griffteil 303 (Pfeil E).

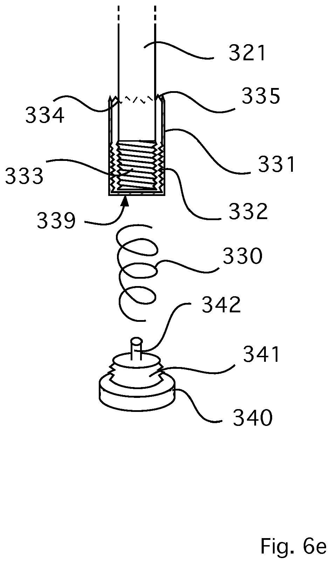

Der erste Abschnitt 322 des zweiten Tragarms 321 ist mit seinem Ende, das dem zweiten Abschnitt 323 abgewandt ist, in eine Hülse 331 eingeschraubt. Die Hülse 331 weist dazu ein Innengewinde 332 auf, in das der erste Abschnitt 322 mit einem Gegengewinde 333, das in dessen Mantelfläche ausgebildet ist, greift (siehe auch Fig. 6d). Am Rand 334 der Hülse 331, der dem Arbeitsende 308 zugewandt ist, sind Zähne 335 ausgebildet, die sich in axialer Richtung erstrecken (siehe Fig. 6e). Die Zähne 335 greifen unter Einwirkung der Feder 330 in korrespondierende Zähne 336 ein, die in der Durchgangsbohrung 307 des zweiten Griffteils 304 ausgebildet sind. Dazu weist die Durchgangsbohrung 307 in dem Abschnitt 337, der an die Stirnseite 309 angrenzt, einen größeren Durchmesser auf, wodurch in der Durchgangsbohrung 307 eine umlaufende Kante 338 entsteht, in der die korrespondierende Zähne 336 ausgebildet sind. Abgesehen von Abschnitt 337 entspricht der Durchmesser der Durchgangsbohrung 307 dem Durchmesser der Durchgangsbohrung 306 im ersten Griffteil 303.

Die Feder 330 drückt gegen den Boden 339 der Hülse 331 (Pfeil E), wodurch die Zähne 335 gegen die korrespondierenden Zähne 336 gepresst werden. Auf diese Weise wird eine unerwünschte Verdrehung des ersten Abschnittes 322 verhindert. Mit der Hülse 331 wird er erste Abschnitt 322 in Richtung des Arbeitsendes 308 gepresst. Die Bewegung des erste Abschnittes 322 in Richtung des Arbeitsendes 308 wird durch das Stellelement 327 begrenzt, das gegen die ihm zugewandte Stirnseite des ersten Griffteiles 303 gepresst wird. Drückt nun der Behandler das Stellelement 327 von der Stirnseite des ersten Griffteils 303 in Richtung der gegenüberliegenden Stirnseite des zweiten Griffteiles 304 (Pfeil F) werden die Zähne 335 der Hülse 331 aus den korrespondierenden Zähnen 336 gelöst, so dass der zweite Tragarm 321 um die Längsachse A des Griffes 302 (Pfeil D) gedreht werden kann. Sobald der Behandler das Ausüben von Druck auf das Stellelement 327 aufgibt, presst die Feder 330 das Stellelement 327 wieder gegen die Stirnseite des ersten Griffteiles 303 und die Zähne 335 der Hülse 331 in die korrespondierenden Zähne 336 in der inneren Mantelfläche des zweiten Griffteils 304. Damit ist der zweite Tragarm 321 wieder in dem Griff 302 fixiert.

Die Drehung des zweiten Tragarmes 321 um die Längsachse A ist mittels der Zähne 335 auf vorgegebene Winkelpositionen beschränkt. Je nach Breite und Höhe der einen Zahnkranz bildenden Zähne 335 können Rastpositionen ausgebildet werden, die einem vorgegebenen Winkelabstand, beispielsweise 90°, 180° oder 270°, ausgehend von der Nullposition entsprechen.

Die Stirnseite 309 des zweiten Griffteiles 304 ist mit einer Kappe 340 verschlossen ist. Dazu ist an der Stirnseite 309 in der inneren Mantelfläche des zweiten Griffteils 304 ein Innengewinde ausgebildet, in das ein von der Kappe 340 abstehender axialer Stift 341, der ein Gegengewinde an seiner Außenseite aufweist, greift. Zwischen der Kappe 340 und dem Boden 339 der Hülse 331, der der Kappe zugewandt ist, ist die Feder 330 angeordnet, die auf einen axialen Vorsprung 342 aufgesteckt ist, der sich von dem Stift 341 in Richtung des Arbeitsendes 308 erstreckt.

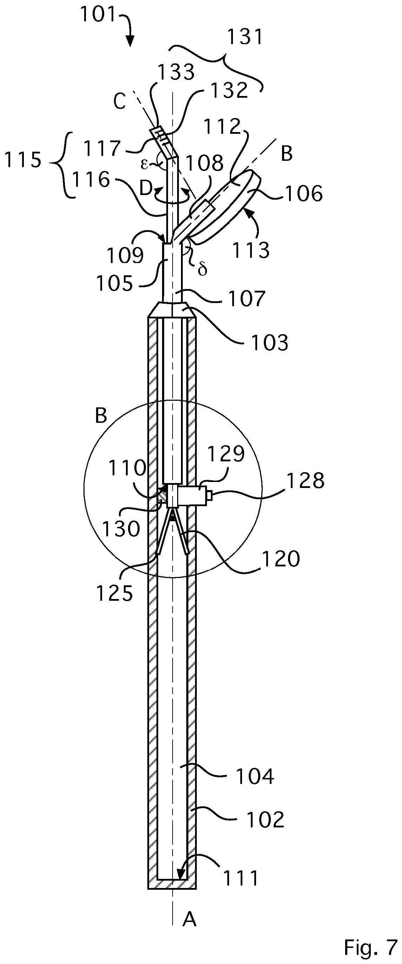

Die in den Figuren 7 bis 8f gezeigte vierte Ausführungsform des erfindungsgemäßen Instrumentes 101 weist einen Griff 102 auf. Der Griff 102 ist ein stielförmiger Körper, dessen Haupterstreckungsrichtung seine Längsachse A ist. An einer Stirnseite des Griffes 102, dem Arbeitsende 103, ist eine Bohrung 104 in den Griff 102 gebracht, die koaxial zur Längsachse A des Griffes 102 verläuft.

Das Instrument 101 weist ferner einen ersten Tragarm 105 auf, der unlösbar mit dem Griff 102 verbunden und an dem der Spiegel 106 befestigt ist. Der erste Tragarm 105 ist insbesondere nicht drehbar, sondern starr in dem Griff 102 befestigt. Der erste Tragarm 105 weist einen ersten Abschnitt 107 und einen an den ersten Abschnitt 107 angrenzenden zweiten Abschnitt 108 auf. Der erste Abschnitt 107 des ersten Tragarms 105 ist ein länglicher Körper mit einem annähernd kreisförmigen Querschnitt. Der erste Abschnitt 107 ist in die Bohrung 104 des Griffes 102 eingeführt und unlösbar mit dem Griff 102 verbunden. Die Haupterstreckungsrichtung des ersten Abschnittes 107, seine Längsachse, liegt dabei auf der Längsachse A des Griffes 102. Der erste Abschnitt 107 erstreckt sich über die Bohrung 104 an dem Arbeitsende 103 des Griffes 102 hinaus, so dass der erste Abschnitt 107 mit einer ersten Stirnseite 109 freiliegt. Die Ausdehnung des ersten Abschnittes 107 in Richtung der Längsachse A innerhalb der Bohrung 104 ist geringer als die Ausdehnung der Bohrung 104 in dieser Richtung, so dass der erste Abschnitt 107 mit seiner zweiten Stirnseite 110 vom Boden 111 der Bohrung 104 beabstandet ist.

Der zweite Abschnitt 108 des Tragarms 105 grenzt mit einer seiner Stirnseiten an die erste Stirnseite 109 des ersten Abschnittes 107 des Tragarms 105 an. Er weist einen geringeren Querschnitt als der erste Abschnitt 107 auf. Der zweite Abschnitt 108 ist zum ersten Abschnitt 107 abgewinkelt. Die Haupterstreckungsrichtung des zweiten Abschnittes 108, seine Längsachse B, verläuft in einem stumpfen Winkel δ zur Längsachse A des Griffes und damit zur Längsachse des ersten Abschnittes 107. An dem zweiten Abschnitt 108 ist der Spiegel 106 mit seiner Rückseite 112 lösbar befestigt. Dabei kann es sich um die Rückwand einer Fassung (nicht gezeigt) oder die Rückseite eines Grundkörpers handeln. Die Vorderseite 113 des Spiegels 106 ist eine reflektierende Oberfläche und damit die eigentliche Spiegelfläche. Die Vorderseite 113 des Spiegels 106 ist dem Griff 102, insbesondere dessen Arbeitsende 103 zugewandt, die Rückseite 112 dem Griff 102 abgewandt. Die vierte Ausführungsform des erfindungsgemäßen Instrumentes 101 ist Fig. 7 als aufrechtstehendes Instrument gezeigt, bei dem die Haupterstreckungsrichtung des Griffes 102, seine Längsachse A, lotrecht verläuft. In dieser Lage des Instrumentes 101 weist die Vorderseite 113 des Spiegels 106 nach unten, die Rückseite 112 hingegen nach oben. Zur lösbaren Befestigung des Spiegels 106 an dem zweiten Abschnitt 108 kann eine starre Metalllasche (nicht gezeigt) vorgesehen sein, mit der der Spiegel 106 direkt auf den zweiten Abschnitt 108 aufgeschoben werden kann. Er ist damit austauschbar.

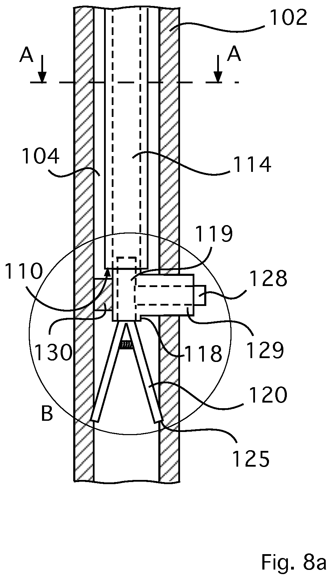

In dem ersten Abschnitt 107 des ersten Tragarms 105 befindet sich eine zylinderförmige Durchgangsbohrung 114 (siehe Fig. 8a), die sich von seiner ersten Stirnseite 109 bis zu dessen zweiter Stirnseite 110 erstreckt. Die Längsachse der Durchgangsbohrung 114 kann koaxial oder achsparallel zur Längsachse des ersten Abschnittes 107 des Tragarms 105 liegen. In der in den Fig. 7 bis 8f gezeigten Variante liegt die Längsachse der Durchgangsbohrung 114 koaxial zur Längsachse des ersten Abschnittes 107 des Tragarms 105 und damit zur Längsachse A des Griffes 102. In die Durchgangsbohrung 114 ist der zweite Tragarm 115 um eine Drehachse drehbar gelagert. Die Drehachse liegt auf der Längsachse der Durchgangsbohrung 114.

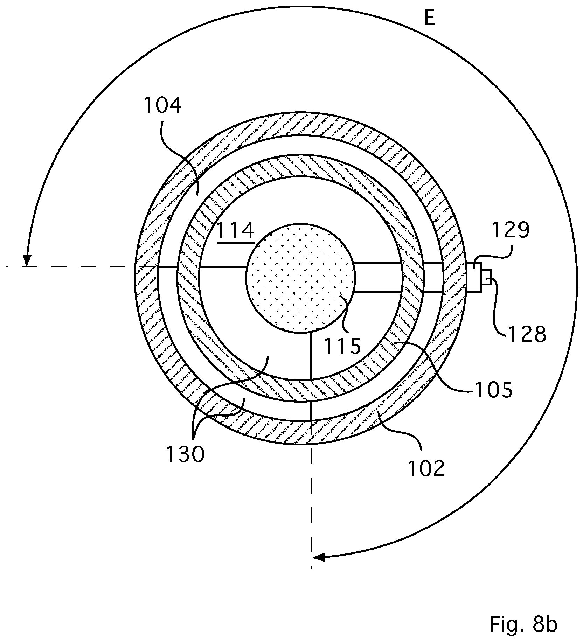

Der zweite Tragarm 115 weist einen ersten Abschnitt 116 und einen an den ersten Abschnitt 116 angrenzenden zweiten Abschnitt 117 auf. Der erste Abschnitt 116 des zweiten Tragarms 115 ist ein länglicher Körper mit einem annähernd kreisförmigen Querschnitt. Der erste Abschnitt 116 ist in der Durchgangsbohrung 114 des ersten Tragarms 105 drehbar gelagert (siehe insbesondere Fig. 8b). Die Haupterstreckungsrichtung des ersten Abschnittes 116, seine Längsachse, liegt dabei auf der Längsachse der Durchgangsbohrung 114. Der erste Abschnitt 116 erstreckt sich über beide Stirnseiten 109, 110 des ersten Abschnittes 107 des ersten Tragarms 105 hinaus. Damit liegt der erste Abschnitt 116 des zweiten Tragarms 115 an beiden Stirnseiten 109, 110 des ersten Abschnittes 107 des ersten Tragarms 105 frei. Dabei ist die Ausdehnung des ersten Abschnittes 116 an der ersten Stirnseite 109 des ersten Tragarms 105 in Richtung der Längsachse A größer als die Ausdehnung des zweiten Abschnittes 108 des ersten Tragarms 105 und des Spiegels 106, so dass eine Drehung des zweiten Tragarmes 115 nicht von dem ersten Tragarm 105 oder dem Spiegel 106 behindert wird.

An der zweiten Stirnseite 110 des ersten Abschnittes 107 des ersten Tragarms 105 steht der erste Abschnitt 116 mit einem Überstand 118 über. Der Überstand 118 erstreckt sich in die Bohrung 104 des Griffes 102, ist aber von deren Boden 111 beabstandet. In den Überstand 118 ist an seiner Stirnseite eine zylinderförmige Bohrung 119, die im Folgenden als Stellbohrung bezeichnet wird, eingebracht. In die Stellbohrung greift ein Fixierungselement 120 zur lösbaren Fixierung des zweiten Tragarms 115 im Griff 102 ein.

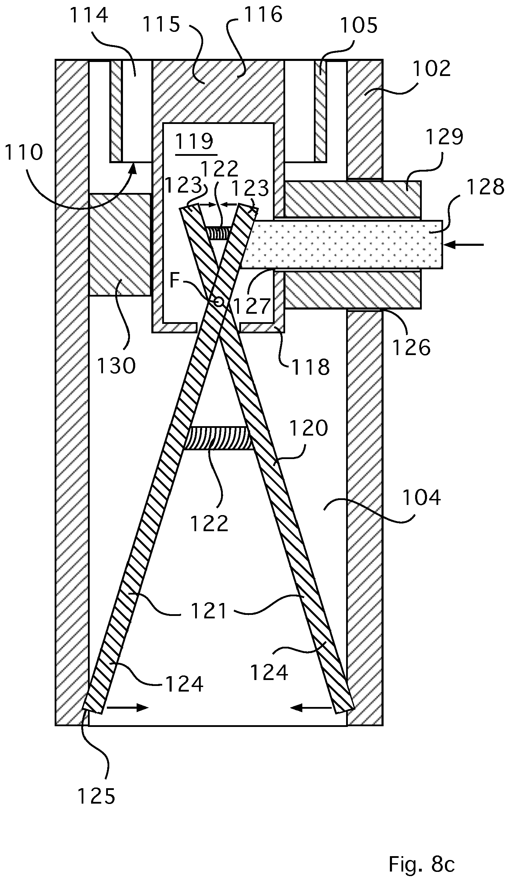

Das Fixierungselement 120 weist zwei Metallstäbe auf (siehe insbesondere Fig. 8c), wobei die Metallstäbe 121 beweglich zueinander unter Erhalt einer X-förmigen Anordnung aneinander gelagert sind und wobei am Kreuzungspunkt der beiden Metallstäbe 121 ein Drehgelenk ausgebildet ist, beispielsweise in Form eines Stiftes oder eine Schraube, die in der Drehachse des Drehgelenkes liegen kann. Das Drehgelenk ermöglicht eine Drehung der Metallstäbe um eine Drehachse F, die orthogonal zur Längsachse A des Griffes 102 liegt. Das Fixierungselement 120 kann in Höhe seiner Drehachse an der Innenwand der Bohrung 119 befestigt sein, ohne dass die Bewegung der Metallstäbe um die Drehachse des Drehgelenkes beeinträchtigt wird. Die Verbindung zwischen beiden Metallstäben ist beispielsweise eine Schraubverbindung.

Zwischen den sich gegenüberliegenden Schenkeln der beiden Metallstäbe 121 ist jeweils eine Feder 122 angeordnet, die die Schenkel der beiden Metallstäbe 121 auseinander pressen und das Fixierungselement 120 gleichzeitig stabilisieren. Ein erstes Paar 123 der sich gegenüberliegenden Schenkel ist in die Stellbohrung 119 des Überstandes 118 eingeführt. Die Feder 122, die zwischen den sich gegenüberliegenden Schenkeln des ersten Paares 123 von Schenkeln angeordnet ist, presst die freien Enden der Schenkel gegen die Wandung der Stellbohrung 119. Das andere, zweite Paar 124 von Schenkeln befindet sich außerhalb der Stellbohrung 119 in der Bohrung 104 des Griffes 102. Die Feder 122, die zwischen den sich gegenüberliegenden Schenkeln des zweiten Paares 124 von Schenkeln angeordnet ist, presst die freien Enden der Schenkel des zweiten Paares 124 gegen die Wandung der Bohrung 104 des Griffes 102. Dort befinden sich in der Wandung der Bohrung 104 Rastkerben 125, in die die freien Enden der Schenkel des zweiten Paares 124 einrasten können. Bei den Rastkerben 125 handelt es sich um scharf begrenzte Vertiefungen, die in der Wandung der Bohrung 104 im Griff 102 ausgebildet sind und sich in Umfangsrichtung der Wandung erstrecken. Die Länge der Rastkerben 125 in Umfangsrichtung ist so ausgebildet, dass eine Drehung des zweiten Tragarms 115 von einer Ausgangsposition um 90° und von dort zurück in die Ausgangsposition möglich ist. Statt mehrerer Rastkerben kann nur eine Rastkerbe vorgesehen sein, die sich über den gesamten Umfang der Wandung erstreckt. Bei den Federn kann es sich beispielsweise um Spreizfedern oder Druckfedern handeln. Das erste Paar 123 sich gegenüberstehender Schenkel kann, wie in Fig. 8c zu erkennen ist, kürzer als das zweite Paar 124 sich gegenüberstehender Schenkel sein, bezogen auf deren Ausdehnung in der Haupterstreckungsrichtung des Griffes 102. Damit liegt der größere Teil des Fixierungselementes 120 außerhalb der Stellbohrung 119.

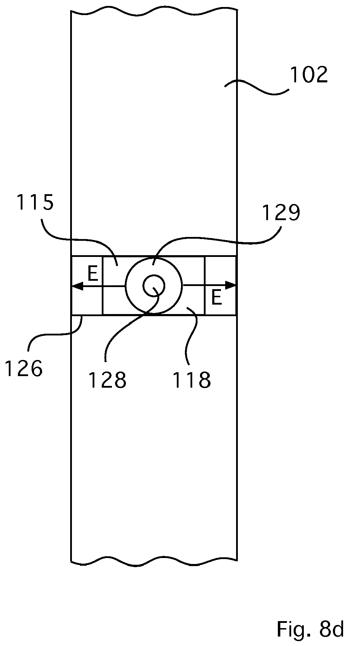

In dem Überstand 118 ist eine Zugangsbohrung 127 zur Stellbohrung 119 in Höhe des ersten Paares 123 sich gegenüberliegender Schenkel ausgebildet. Die Längsachse der Zugangsbohrung 127 verläuft ebenfalls radial zur Längsachse A des Griffes 102. In der Höhe der Zugangsbohrung 127 befindet sich in dem Griff 102 eine schlitzartige Öffnung 126 (siehe insbesondere Fig. 8d). Von der Zugangsbohrung 127 erstreckt sich eine Führungshülse 129, deren Längsachse koaxial zur Längsachse der Zugangsbohrung 127 ist, durch die Öffnung 126, die in dem Griff 102 ausgebildet ist, aus der Bohrung 104 hinaus. Die Führungshülse 129 ist fest an dem Überstand 118 angebracht und steht in radialer Richtung zur Längsachse A an der Griffaußenseite über. Durch die Führungshülse 129 und die Zugangsbohrung 127 ist ein Stift 128 geführt. Der Stift 128 ist mit dem ihm nächstliegenden Schenkel des ersten Paares 123 von Schenkeln verbunden. Die Längsachse des Stiftes 128 liegt dabei radial zur Längsachse A des Griffes 102 und ebenso orthogonal zur Drehachse des Fixierungselementes 120.

Die schlitzartige Öffnung 126 ist ein Langloch, dessen Längskanten sich in Umfangsrichtung des Griffes 102 erstrecken. Die Ausdehnung des Langloches in Umfangsrichtung ist so gewählt, dass der zweite Tragarm 115 bis zu 270° gedreht werden kann (Pfeil D in Fig. 7). In Umfangsrichtung des Griffes 102 kann sich daher die Längskanten der Öffnung 126 beispielsweise über einen Kreisbogen von 270 ° erstrecken. Innerhalb des Langloches kann somit die Führungshülse 129 mit dem Stift 128, sofern dieser nach innen gegen das erste Paar 123 von sich gegenüberliegenden Schenkeln der Metallstäbe 121 gedrückt ist, bewegt werden (Pfeil E in Fig. 8b).

Mittels des Stiftes 128 kann von außen Druck auf das erste Paar 123 von sich gegenüberliegenden Schenkeln der Metallstäbe 121 ausgeübt werden. Wird Druck auf das erste Paar 123 ausgeübt, wird die Rastverbindung zwischen den freien Enden der Schenkel des zweiten Paares 124 von sich gegenüberliegenden Schenkeln der Metallstäbe 121 gelöst. Damit kann der zweite Tragarm 115 um Winkelabstände von 90°, 180° oder 270°, bezogen auf eine Nullposition, um die Längsachse A gedreht werden (in Richtung des Pfeiles D in Fig. 7), in dem die Führungshülse 129 entlang Pfeil E bewegt wird. Sobald über den Stift 128 kein Druck mehr ausgeübt wird, werden die freien Enden der Schenkel des zweiten Paares 124 von sich gegenüberliegenden Schenkeln der Metallstäbe 121 wieder gegen die Wandung der Bohrung 104 gepresst, und zwar mittels der zwischen ihnen liegenden Feder 122. Sie rasten dann erneut in die Rastkerben 125 ein.

In der Bohrung 104 kann in dem Bereich, der der Zugangsbohrung 127 und der Führungshülse 129, bezogen auf deren Längsachse, gegenüberliegt, ein Gegenstück 130 angeordnet sein. Das Gegenstück 130 ist so angeordnet, dass Druck, der mittels des Stiftes 128 auf das Fixierungselement 120 ausgeübt wird, nicht zur Verschiebung des ersten Abschnittes 116 des zweiten Tragarms 115 führt. Das Gegenstück 130 ist an der Wandung der Bohrung 104 befestigt und erstreckt sich in radialer Richtung, bezogen auf die Längsachse A, bis zum Überstand 118, ohne an diesem befestigt zu sein.

Der zweite Abschnitt 117 des zweiten Tragarms 115 ist mit seiner Haupterstreckungsrichtung (Längsachse C in Fig. 7) in einem stumpfen Winkel ε abgewinkelt zur Hauptstreckungsrichtung, dessen Längsachse, des ersten Abschnittes 116 des zweiten Tragarmes 115 ausgebildet. Der zweite Abschnitt 117 kann ein länglicher Körper sein. Der zweite Abschnitt 117 bildet das Positionierungselement 131. Er weist dazu die Längenmesseinrichtung 132 des Positionierungselementes 131, eine Skala, auf. Je nach geometrischer Form des zweiten Abschnittes 117 bilden dessen Oberflächen, beispielsweise dessen Flächenseiten, Kanten und/oder Stirnseiten, die Kontakteinrichtung 133 des Positionierungselementes 131. Ist der zweite Abschnitt 117 ein flacher Körper, so bilden die Kanten des zweiten Abschnittes 117 und seine Flächenseiten die Kontakteinrichtung 133. Das ist bei dem Werkzeug eines Heidemann-Spatels der Fall. Ist der zweite Abschnitt 117 ein stiel- oder stangenförmiger Körper, so bilden die Mantelfläche des zweiten Abschnittes 117 und seine Spitze oder abgerundete oder nicht-abgerundete Stirnseite die Kontakteinrichtung 133. Das ist bei dem Werkzeug einer Sonde der Fall.

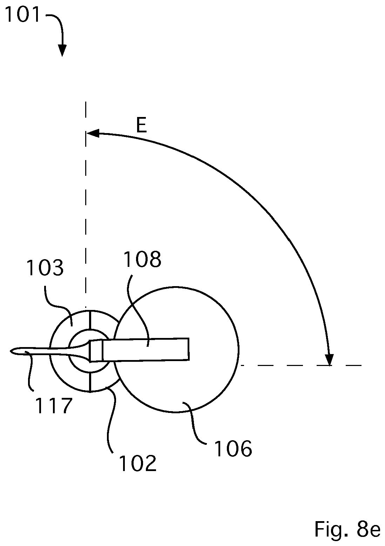

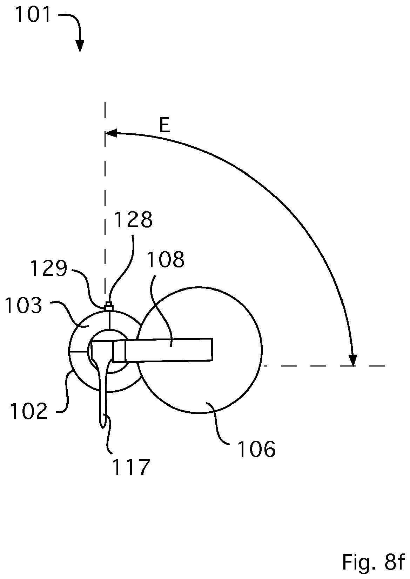

In Ausgangsposition (siehe Fig. 8e) verlaufen die Haupterstreckungsrichtung (Längsachse C in Fig. 7) des zweiten Abschnittes 117 des zweiten Tragarms 115, die Haupterstreckungsrichtung (Längsachse B in Fig. 7) des zweiten Abschnittes 108 des ersten Tragarms 105 und die Längsachse A des Griffes 102 in einer ersten Ebene. Durch Drücken des Stiftes 128 auf das Fixierungselement 120 und Verschieben der Führungshülse 129 samt des Stiftes 128 in der schlitzartigen Öffnung 126 wird der zweite Tragarm in diesem Beispiel um 90° gedreht, wodurch die in Fig. 8f gezeigte Position erreicht wird. Drehungen um 180° oder 270° sind ebenso möglich. In der in Fig. 8f gezeigten Position liegt die Haupterstreckungsrichtung (Längsachse C in Fig. 7) des zweiten Abschnittes 117 des zweiten Tragarms 115 in einer zweiten Ebene, die orthogonal zur ersten Ebene verläuft. Die Längsachse A des Griffes 102 liegt in beiden Ebenen.

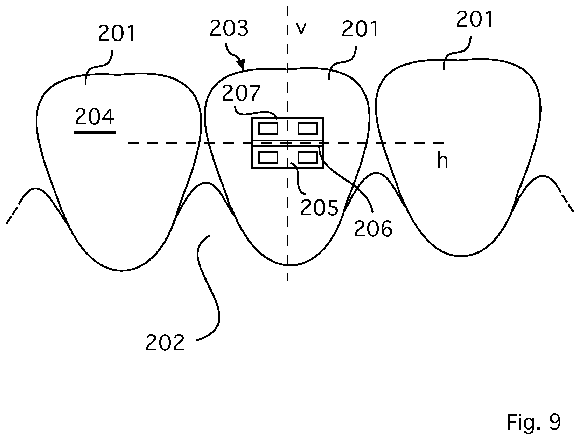

Fig. 9 veranschaulicht die Positionierung eines Brackets auf der Oberfläche eines Zahnes. Gezeigt sind drei Schneidezähne, die sich am Unterkiefer befinden. Sichtbarer Teil der Zähne sind die klinischen Kronen 201, die über dem Zahnfleisch 202 freiliegen. Die klinischen Kronen 201 erstrecken sich im Mund des Patienten vom Zahnfleisch 202 bis zu ihren Inzisalkanten 203. In Fig. 9 sind die labialen Flächenseiten 204 der klinischen Kronen 201 gezeigt.

Nach der Vorbehandlung der Flächenseite 204, auf die ein Bracket 205 aufgebracht werden soll, und dem Auftragen des Haftvermittlers auf die Flächenseite 204 wird das auf seiner Rückseite mit dem Adhäsiv versehene Bracket 205 vom Behandler mittels einer Pinzette auf die Flächenseite 204 aufgebracht, so dass das Bracket 205 mit seiner Rückseite an der Flächenseite 204 anliegt. Der Behandler nimmt dabei eine grobe Vorpositionierung des Brackets 205 vor. Das Bracket weist an seiner Vorderseite einen Schlitz, den Bracketslot 206, auf. Die der Inzisalkante 203 zugewandte Kante des Bracket 205 ist dessen okklusale Bracketkante 207.

Nach dem Aufbringen des Brackets 205 auf die Flächenseite 204 wird zur exakten Positionierung des Brackets 205 mittels des erfindungsgemäßen Instrumentes 1, 101, 301 folgendermaßen vorgegangen:
(i) Die Neigung bzw. Angulation des Brackets 205 wird mittels der Kontakteinrichtung 14, 133, 326 des Positionierungselementes 6, 131, 324 korrigiert und überschüssiges Adhäsiv mittels der Kontakteinrichtung 14, 133, 326 des Positionierungselementes 6, 131 entfernt.
(ii) Vertikale Korrektur: Dazu wird die Brackethöhe und damit die vertikale Positionierung des Brackets auf der Flächenseite 204 überprüft (Achse v in Fig. 9) und, falls die Brackethöhe nicht den Vorgaben entspricht, korrigiert. Die Brackethöhe ist im Falle eines Schneidezahnes der Abstand des Bracketslots 206 von der Inzisalkante 203 des Zahnes oder der Abstand der okklusalen Bracketkante 207 von der Inzisalkante 203 des Zahnes. Die Brackethöhe wird mit der Längenmesseinrichtung 13, 132, 325 des Positionierungselementes 6, 131, 324 gemessen und, wenn die Messung ergibt, dass die tatsächliche Brackethöhe nicht der erwünschten Brackethöhe entspricht, mittels der Kontakteinrichtung 14, 133, 326 des Positionierungselementes 6, 131, 324 in vertikaler Richtung (Achse v) verschoben. Dazu kann die Kontakteinrichtung 14, 133, 324 beispielsweise in den Bracketslot 206 eingelegt werden. Anschließend wird die Brackethöhe erneut mit der Längenmesseinrichtung 13, 132 des Positionierungselementes 6, 131 gemessen, und, falls erforderlich, eine weitere Korrektur vorgenommen. Dieser Vorgang wird wiederholt, bis das Bracket 205 die exakte Brackethöhe aufweist.
(iii) Horizontale Korrektur: Dazu wird die mesio-distale Position und damit die horizontale Positionierung des Brackets 205 mittels des Spiegels 5, 106, 314 überprüft (Achse h in Fig. 9). Wenn die Messung ergibt, dass die tatsächliche mesio-distale Position des Brackets 205 nicht der erwünschten Brackethöhe entspricht, wird das Bracket mittels der Kontakteinrichtung 14, 133, 326 des Positionierungselementes 6, 131, 324 in horizontaler Richtung (Achse h) verschoben. Anschließend wird die mesio-distale Position erneut mittels des Spiegels 5, 106, 314 überprüft und, falls erforderlich, eine weitere Korrektur vorgenommen. Dieser Vorgang wird wiederholt, bis das Bracket 205 die exakte mesio-distale Position aufweist.

### Bezugszeichenliste

1 Instrument; 2 Griff; 3 Arbeitsende des Griffes; 4 Tragarm; 5 Spiegel; 6 Positionierungselement; 7 erster Abschnitt des Tragarms; 8 zweiter Abschnitt des Tragarms; 9 Vorderseite des Spiegels; 10 Rückseite des Spiegels; 11 erstes Segment des Positionierungselementes; 12 zweites Segment des Positionierungselementes; 13 Längenmesseinrichtung; 14 Kontakteinrichtung; 15 dritter Abschnitt des Tragarms;
101 Instrument; 102 Griff; 103 Arbeitsende des Griffes; 104 Bohrung; 105 erster Tragarm; 106 Spiegel; 107 erster Abschnitt des ersten Tragarms; 108 zweiter Abschnitt des ersten Tragarms; 109 erste Stirnseite des ersten Abschnittes 107; 110 zweite Stirnseite des ersten Abschnittes 107; 111 Boden der Bohrung; 112 Rückseite des Spiegels; 113 Vorderseite des Spiegels; 114 Durchgangsbohrung; 115 zweiter Tragarm; 116 erster Abschnitt des zweiten Tragarms; 117 zweiter Abschnitt des zweiten Tragarms; 118 Überstand; 119 Stellbohrung; 120 Fixierungselement; 121 Metallstab; 122 Feder; 123 erstes Paar sich gegenüberliegender Schenkel; 124 zweites Paar sich gegenüberliegender Schenkel; 125 Rastkerbe; 126 Öffnung; 127 Zugangsbohrung; 128 Stift; 129 Führungshülse; 130 Gegenstück; 131 Positionierungselement; 132 Längenmesseinrichtung; 133 Kontakteinrichtung;
201 klinische Krone; 202 Zahnfleisch; 203 Inzisalkante; 204 Flächenseite; 205 Bracket; 206 Bracketslot; 207 okklusale Bracketkante;
301 Instrument; 302 Griff; 303 erstes Griffteil; 304 zweites Griffteil; 305 Steg; 306 Durchgangsbohrung; 307 Durchgangsbohrung; 308 Arbeitsende; 309 Stirnseite; 310 Spalt; 311 Ausnehmung; 312 Schraube; 313 erster Tragarm; 314 Spiegel; 315 erster Abschnitt; 316 zweiter Abschnitt; 317 Stirnseite; 318 Vorderseite des Spiegels; 319 Rückseite des Spiegels; 320 Durchgangsbohrung; 321 zweiter Tragarm; 322 erster Abschnitt; 323 zweiter Abschnitt; 324 Positionierungselement; 325 Längenmesseinrichtung; 326 Kontakteinrichtung; 327 Stellelement; 328 Führungsloch; 329 äußerer Rand; 330 Feder; 331 Hülse; 332 Innengewinde; 333 Gegengewinde; 334 Rand; 335 Zähne; 336 korrespondierende Zähne; 337 Abschnitt; 338 Kante; 339 Boden; 340 Kappe; 341 Stift; 342 Vorsprung.

## Patentansprüche

1. Instrument für zahnärztliche Zwecke, insbesondere zur Positionierung von Brackets (205) an Zähnen, wobei das Instrument (1, 101, 301) einen Spiegel (5, 106, 314) und ein Positionierungselement (6, 131, 324) zur Bestimmung und Veränderung der Position des Brackets (205) am Zahn aufweist, wobei das Instrument (1, 101, 301) einen Griff (2, 102, 302) aufweist und wobei der Spiegel (5, 106, 314) und das Positionierungselement (6, 131, 324) am selben Ende des Griffes (2, 102, 302) angeordnet sind, **dadurch gekennzeichnet, dass** das Positionierungselement (6) ein erstes Segment (11) und ein zweites Segment (12) aufweist, und dass die Haupterstreckungsrichtung des zweiten Segmentes (12) annähernd parallel zur Längsachse (A) des Griffes (2) verläuft.

2. Instrument nach Anspruch 1, **dadurch gekennzeichnet, dass** der Griff (2, 102, 302) einen zylinderförmigen oder annähernd zylinderförmigen Grundkörper aufweist.

3. Instrument nach Anspruch 1 oder Anspruch 2, **dadurch gekennzeichnet, dass** der Spiegel (5, 106, 314) zur Haupterstreckungsrichtung des Griffes (2, 102, 302) abgewinkelt angeordnet ist.

4. Instrument nach einem der vorstehenden Ansprüche, **dadurch gekennzeichnet, dass** das Positionierungselement (6, 131, 324) eine Längenmesseinrichtung (13, 132, 325) zur Bestimmung der Position des Brackets (205) am Zahn aufweist.

5. Instrument nach einem der vorstehenden Ansprüche, **dadurch gekennzeichnet, dass** das Positionierungselement (6, 131, 324) eine Kontakteinrichtung (14, 133, 326) zur Kontaktierung des Brackets (205) und zur Veränderung der Position des Brackets (205) am Zahn aufweist

6. Instrument nach einem der vorstehenden Ansprüche, **dadurch gekennzeichnet, dass** es einen Tragarm (4) mit einem ersten Abschnitt (7) aufweist, über den der Tragarm (4) an dem Arbeitsende (3) des Griffs (2) lösbar oder unlösbar befestigt ist, wobei der Tragarm (4) einen zweiten Abschnitt (8) aufweist, dessen Haupterstreckungsrichtung in einem stumpfen Winkel α zur Hauptstreckungsrichtung des Griffes (2) verläuft, und an dem der Spiegel (5) unlösbar oder lösbar befestigt ist.

7. Instrument nach Anspruch 6, **dadurch gekennzeichnet, dass** der Tragarm (4) einen dritten Abschnitt (15) aufweist, dessen Haupterstreckungsrichtung abgewinkelt zum zweiten Abschnitt (8) des Tragarmes (4) ist, wobei der dritte Abschnitt (15) das Positionierungselement (6) ganz oder teilweise bildet oder das Positionierungselement (6) an dem dritten Abschnitt (15) befestigt ist.

8. Instrument nach Anspruch 7, **dadurch gekennzeichnet, dass** sich der dritte Abschnitt (15) mit seiner Haupterstreckungsrichtung in einer Richtung erstreckt, die, bezogen auf den zweiten Abschnitt (8) des Tragarmes (4), der Haupterstreckungsrichtung des ersten Abschnittes (7) abgewandt ist.

9. Instrument nach einem der vorstehenden Ansprüche, **dadurch gekennzeichnet, dass** das Positionierungselement (6) abgewinkelt zur Haupterstreckungsrichtung des Spiegels (5) angeordnet ist und sich in eine Richtung erstreckt, die dem Griff (2) abgewandt ist.

10. Instrument nach einem der Ansprüche 1 bis 5, **dadurch gekennzeichnet, dass** es aufweist:
- einen ersten Tragarm (105, 313) mit einem ersten Abschnitt (107, 315), über den der erste Tragarm (105, 313) an dem Arbeitsende (103, 308) des Griffs (102, 302) befestigt ist, wobei der erste Tragarm (105, 313) einen zweiten Abschnitt (108, 316) aufweist, dessen Haupterstreckungsrichtung in einem stumpfen Winkel δ zur Hauptstreckungsrichtung des Griffes (102, 302) verläuft, und an dem der Spiegel (106, 314) lösbar befestigt ist; und
- einen zweiten Tragarm (115, 321) mit einem ersten Abschnitt (116, 322), über den der zweite Tragarm (115, 321) an dem Arbeitsende (103, 308) des Griffs (102, 302) oder an dem ersten Tragarm (105, 321) befestigt ist, wobei der zweite Tragarm (115, 321) einen zweiten Abschnitt (117, 323) aufweist, dessen Haupterstreckungsrichtung in einem stumpfen Winkel ε zur Hauptstreckungsrichtung des Griffes (102, 302) verläuft.

11. Instrument nach Anspruch 10, **dadurch gekennzeichnet, dass** der zweite Tragarm (115, 321) um eine Drehachse drehbar an dem Arbeitsende (103, 308) des Griffs (102, 302) oder an dem ersten Tragarm (105, 313) befestigt ist, wobei die Drehachse koaxial oder achsparallel zur Hauptstreckungsrichtung des Griffes (102, 302) verläuft.

12. Instrument nach Anspruch 10 oder Anspruch 11, **dadurch gekennzeichnet, dass** der erste Abschnitt (107, 315) des ersten Tragarmes (105, 313) eine Bohrung (114, 320) zur Lagerung des zweiten Tragarmes (115, 321) in dem ersten Tragarm (105, 313) aufweist.

13. Instrument nach Anspruch 12, **dadurch gekennzeichnet, dass** der erste Abschnitt (107) des ersten Tragarms (105) ein an seinen beiden Stirnseiten (109, 110) offener Hohlzylinder ist, und der erste Abschnitt (116) des zweiten Tragarmes (115) durch den ersten Abschnitt (107) des ersten Tragarmes (105) geführt ist, wobei der erste Abschnitt (116) des zweiten Tragarmes (115) innerhalb des Griffes (102) mit einem Überstand (118) aus dem ersten Abschnitt (107) des ersten Tragarmes (105) heraussteht und in den Überstand (118) ein Stellelement (128) eingreift, das eine Drehung des zweiten Tragarmes (115) ermöglichen kann.

14. Instrument nach Anspruch 13, **dadurch gekennzeichnet, dass** der Überstand (118) an seiner dem ersten Tragarm (105) abgewandten Stirnseite eine Bohrung (119) aufweist, in die ein Fixierungselement (120) zur lösbaren Fixierung des zweiten Tragarmes (115) in dem Griff (102) eingreift.

15. Instrument nach einem der vorstehenden Ansprüche, **dadurch gekennzeichnet, dass** der Griff (302) ein erstes Griffteil (303) mit einer Durchgangsbohrung (306) und ein zweites Griffteil (304) mit einer Bohrung (307) aufweist, die axial zur Haupterstreckungsrichtung des Griffes (302) verlaufen, wobei die Durchgangsbohrung (306) des ersten Griffteils (303) und die Bohrung (307) des zweiten Griffteils (304) fluchtend zu einander verlaufen, wobei
- der erste Abschnitt (322) des zweiten Tragarms (321) durch die Durchgangsbohrung (306) des ersten Griffteiles (303) in die Bohrung (307) des zweiten Griffteiles (304) geführt und dort drehbar gelagert ist;
- der erste Griffteil (303) und der zweite Griffteil (304) über ein Verbindungsmittel (305) unter Ausbildung eines Spaltes (310) zwischen den einander zugewandten Stirnseiten des ersten und zweiten Griffteils (303, 304) miteinander verbunden sind; und
- ein Stellelement (327) an dem ersten Abschnitt (322) des zweiten Tragarms (321) ausgebildet ist, das sich in den Spalt (310) erstreckt und mit dem eine Drehung des zweiten Tragarmes (321) bewirkt werden kann.

## Claims

1. An instrument for dental use, especially for positioning brackets (205) at teeth, wherein the instrument (1, 101, 301) has a speculum (5, 106, 314) and a positioning element (6, 131, 324) for determining and changing the position of the bracket (205) at the tooth, wherein the instrument (1, 101, 301) has a handle (2, 102, 302) and wherein the speculum (5, 106, 314) and the positioning element (6, 131, 324) are arranged on the same end of the handle (2, 102, 302), **characterized in that** the positioning element (6) has a first segment (11) and a second segment (12), and that the main extension direction of the second segment (12) extends approximately in parallel to the longitudinal axis (A) of the handle (2).

2. The instrument according to claim 1, **characterized in that** the handle (2, 102, 302) has a cylindric or approximately cylindric main body.

3. The instrument according to claim 1 or claim 2, **characterized in that** the speculum (5, 106, 314) is arranged at an angle to the main extension direction of the handle (2, 102, 302).

4. The instrument according to any of the preceding claims, **characterized in that** the positioning element (6, 131, 324) has a length measuring means (13, 132, 325) for determining the position of the bracket (205) at the tooth.

5. The instrument according to any of the preceding claims, **characterized in that** the positioning element (6, 131, 324) has a contacting means (14, 133, 326) for contacting the bracket (205) and for changing the position of the bracket (205) at the tooth.

6. The instrument according to any of the preceding claims, **characterized in that** it has a supporting arm (4) having a first portion (7) via which the supporting arm (4) is detachably or permanently attached to the working end (3) of the handle (2), wherein the supporting arm (4) has a second portion (8) the main extension direction of which extends at an obtuse angle α to the main extension direction of the handle (2) and to which the speculum (5) is permanently or detachably attached.

7. The instrument according to claim 6, **characterized in that** the supporting arm (4) has a third portion (15) the main extension direction of which is at an angel to the second portion (8) of the supporting arm (4), wherein the third portion (15) completely or partially forms the positioning element (6) or the positioning element (6) is attached to the third portion (15).

8. The instrument according to claim 7, **characterized in that** the third portion (15) with its main extension direction extends in a direction facing away from the main extension direction of the first portion (7) with respect to the second portion (8) of the supporting arm (4).

9. The instrument according to any of the preceding claims, **characterized in that** the positioning element (6) is arranged at an angle to the main extension direction of the speculum (5) and extends in a direction facing away from the handle (2).

10. The instrument according to any one of claims 1 to 5, **characterized in that** is has:
- a first supporting arm (105, 313) having a first portion (107, 315) via which the first supporting arm (105, 313) is attached to the working end (103, 308) of the handle (102, 302), wherein the first supporting arm (105, 313) has a second portion (108, 316) the main extension direction of which extends at an obtuse angle δ to the main extension direction of the handle (102, 302) and to which the speculum (106, 314) is detachably attached; and
- a second supporting arm (115, 321) having a first portion (116, 322) via which the second supporting arm (115, 321) is attached to the working end (103, 308) of the handle (102, 302) or to the first supporting arm (105, 321), wherein the second supporting arm (115, 321) has a second portion (117, 323) the main extension direction of which extends at an obtuse angle ε to the main extension direction of the handle (102, 302).

11. The instrument according to claim 10, **characterized in that** the second supporting arm (115, 321) is attached to the working end (103, 308) of the handle (102, 302) rotatably about a rotational axis or to the first supporting arm (105, 313), wherein the rotational axis extends coaxially or axially parallel to the main extension direction of the handle (102, 302).

12. The instrument according to claim 10 or claim 11, **characterized in that** the first portion (107, 315) of the first supporting arm (105, 313) has a bore (114, 320) for bearing the second supporting arm (115, 321) in the first supporting arm (105, 313).

13. The instrument according to claim 12, **characterized in that** the first portion (107) of the first supporting arm (105) is a hollow cylinder being open at both of its front sides (109, 110), and the first portion (116) of the second supporting arm (115) is guided through the first portion (107) of the first supporting arm (105), wherein the first portion (116) of the second supporting arm (115) within the handle (102) projects from the first portion (107) of the first supporting arm (105) with a protrusion (118) and an adjusting element (128) engages the protrusion (118) what can enable the second supporting arm (115) to be rotated.

14. The instrument according to claim 13, **characterized in that** the protrusion (118) at its front side facing away from the first supporting arm (105) has a bore (119), which is engaged by a fixation element (120) for detachably fixing the second supporting arm (115) in the handle (102).

15. The instrument according to any of the preceding claims, **characterized in that** the handle (302) has a first handle part (303) with a through bore (306) and a second handle part (304) with a bore (307) that extend axially to the main extension direction of the handle (302), wherein the through bore (306) of the first handle part (303) and the bore (307) of the second handle part (304) are aligned with one another, wherein
- the first portion (322) of the second supporting arm (321) is guided through the through bore (306) of the first handle part (303) into the bore (307) of the second handle part (304) and is rotatably borne there;
- the first handle part (303) is connected to the second handle part (304) via a connecting means (305) to form a gap (310) between the facing front sides of the first and second handle parts (303, 304); and
- an adjusting element (327) is formed on the first portion (322) of the second supporting arm (321) that extends into the gap (310) and with which it is possible to cause a rotation of the second supporting arm (321).

## Revendications

1. Instrument à but orthodontique, en particulier pour le positionnement de brackets (205) sur les dents, l'instrument (1, 101, 301) présentant un miroir (5, 106, 314) et un élément de positionnement (6, 131, 324) pour déterminer et modifier la position du bracket (205) sur la dent, l'instrument (1, 101, 301) présentant une poignée (2, 102, 302), le miroir (5, 106, 314) et l'élément de positionnement (6, 131, 324) étant disposés sur la même extrémité de la poignée (2, 102, 302), cararactérisé par le fait que l'élément de positionnement (6) présente un premier segment (11) et un second segment (12), et que le sens principal d'extension du second segment (12) s'étend de façon sensiblement parallèle à l'axe longitudinal (A) de la poignée (2).

2. Instrument selon la revendication 1, **caractérisé par le fait que** la poignée (2, 102, 302) présente un corps de base cylindrique ou approximativement cylindrique.

3. Instrument selon la revendication 1 ou la revendication 2, **caractérisé par le fait que** le miroir (5, 106, 314) est coudé par rapport au sens principal d'extension de la poignée (2, 102, 302).

4. Instrument selon une des revendications précédentes, **caractérisé par le fait que** l'élément de positionnement (6, 131, 324) présente un dispositif de mesure de longueur (13, 132, 325) pour déterminer la position du bracket (205) sur la dent.

5. Instrument selon une des revendications précédentes, **caractérisé par le fait que** l'élément de positionnement (6, 131, 324) présente un dispositif de contact (14, 133, 326) pour créer le contact du bracket (205) et pour modifier la position du bracket (205) sur la dent.

6. Instrument selon une des revendications précédentes, **caractérisé par le fait qu'**il présente un bras de maintien (4) avec une première partie (7) par laquelle le bras de maintien (4) est fixé à l'extrémité de travail (3) de la poignée (2) de façon amovible ou non-amovible, le bras de maintien (4) présentant une deuxième partie (8) dont le sens principal d'extension s'étend selon un angle obtus α par rapport au sens principal d'extension de la poignée (2), et est fixé au miroir (5) de façon non-amovible ou amovible.

7. Instrument selon la revendication 6, **caractérisé par le fait que** le bras de maintien (4) présente une troisième partie (15) dont le sens principal d'extension est coudé par rapport à la deuxième partie (8) du bras de maintien (4), la troisième partie (15) représentant totalement ou en partie l'élément de positionnement (6) ou que l'élément de positionnement (6) est fixé sur la troisième partie (15).

8. Instrument selon la revendication 7, **caractérisé par le fait que** la troisième partie (15) dans son sens principal d'extension s'étend dans une direction qui, par rapport à la deuxième partie (8) du bras de maintien (4), est opposée au sens principal d'extension de la première partie (7).

9. Instrument selon une des revendications précédentes, **caractérisé par le fait que** l'élément de positionnement (6) est disposé coudé par rapport au sens principal d'extension du miroir (5) et s'étend dans une direction opposée à celle de la poignée (2).

10. Instrument selon une des revendications 1 à 5, **caractérisé par le fait qu'**il présente:
- un premier bras de maintien (105, 313) avec une première partie (107, 315), par laquelle le premier bras de maintien (105, 313) est fixé à l'extrémité de travail (103, 308) de la poignée (102, 302), le premier bras de maintien (105, 313) présentant une deuxième partie (108, 316) dont le sens principal d'extension s'étend selon un angle obtus δ par rapport au sens principal d'extension de la poignée (102, 302) et est fixé au miroir (106, 314) de façon amovible; et
- un deuxième bras de maintien (115, 321) avec une première partie (116, 322), par laquelle le deuxième bras de maintien (115, 321) est fixé à l'extrémité de travail (103, 308) de la poignée (102, 302) ou au premier bras de maintien (105, 321), le deuxième bras de maintien (115, 321) présentant une deuxième partie (117, 323) dont le sens principal d'extension s'étend selon un angle obtus ε par rapport aux sens principal d'extension de la poignée (102, 302).

11. Instrument selon la revendication 10, **caractérisé par le fait que** le deuxième bras de maintien (115, 321) est fixé de façon rotative par un axe de pivotement sur l'extrémité de travail (103, 308) de la poignée (102, 302) ou sur le premier bras de maintien (105, 313), l'axe de pivotement s'étendant de façon coaxiale ou parallèle à l'axe par rapport au sens principal d'extension de la poignée (102, 302).

12. Instrument selon la revendication 10 ou la revendication 11, **caractérisé par le fait que** la première partie (107, 315) du premier bras de maintien (105, 313) présente un perçage (114, 320) pour le logement du deuxième bras de maintien (115, 321) dans le premier bras de maintien (105, 313).

13. Instrument selon la revendication 12, **caractérisé par le fait que** la première partie (107) du premier bras de maintien (105) est, à ses deux extrémités (109, 110), un cylindre creux ouvert, et que la première partie (116) du deuxième bras de maintien (115) est conduit à travers la première partie (107) du premier bras de maintien (105), la première partie (116) du deuxième bras de maintien (115) faisant saillie de l'intérieur de la poignée (102) avec un dépassement (118) sortant de la première partie (107) du premier bras de maintien (105), et que dans ce dépassement (118) vient s'enclencher un élément de réglage (128) rendant possible la rotation du deuxième bras de maintien (115).

14. Instrument selon la revendication 13, **caractérisé par le fait que** le dépassement (118) présente sur l'extrémité opposée au premier bras de maintien (105) un perçage (119), dans lequel vient s'introduire un élément de fixation (120) pour la fixation amovible du deuxième bras de maintien (115) dans la poignée (102).

15. Instrument selon une des revendications précédentes, **caractérisé par le fait que** la poignée (302) présente un premier endroit de prise (303) avec un trou de passage (306) et un deuxième endroit de prise (304) avec un perçage (307), lesquels s'étendant de façon axiale dans le sens principal d'extension de la poignée (302), le trou de passage (306) du premier endroit de prise (303) et le perçage (307) du deuxième endroit de prise (304) sont alignés entre eux, étant donné que :
- la première partie (322) du deuxième bras de maintien (321) est conduite à travers le trou de passage (306) du premier endroit de prise (303) dans le perçage (307) du deuxième endroit de prise (304) et y est fixée de façon pivotante;
- le premier endroit de prise (303) est relié au deuxième endroit de prise (304) par un moyen de liaison (305) en formant une fente (310) entre les extrémités en regard du premier et du deuxième endroit de prise (303, 304); et
- un élément de réglage (327) est réalisé dans la première partie (322) du deuxième bras de maintien, (321), qui s'étend dans la fente (310) et avec lequel peut être effectuée une rotation du deuxième bras de maintien (321).
